# EUROPÄISCHE PATENTSCHRIFT

(11) **EP 0 054 511 B1**
(45) Veröffentlichungstag und Bekanntmachung des Hinweises auf die Patenterteilung: **13.05.1987**
(21) Anmeldenummer: 81810482.0
(22) Anmeldetag: 07.12.1981
(51) Int. Cl.: C07C 121/66, C07C 121/76, C07C 69/773, D06L 3/12, C08K 5/00

(54) **4-Styryl-4'-vinylbiphenyle, Verfahren zu deren Herstellung und deren Verwendung als optische Aufheller**
4-Styryl-4'-vinylbifenyls, methods for their preparation and their use as optical whiteners
4-Styryle-4'-vinylbiphényles, méthodes pour leur préparation et leur emploi comme azurants optiques

(30) Priorität: 12.12.1980 CH 9192/80
(43) Veröffentlichungstag der Anmeldung: 23.06.1982
(73) Patentinhaber: CIBA-GEIGY AG, 4002 Basel (CH)
(72) Erfinder: Guglielmetti, Leonardo, Dr., CH-4103 Bottmingen (CH)

## Beschreibung

Die vorliegende Erfindung betrifft neue 4-Styryl-4'-vinylbiphenyle, Verfahren zu deren Herstellung und deren Verwendung zum optischen Aufhellen von synthetischen, halbsynthetischen und natürlichen hochmolekularen organischen Materialien.

Aus der DE-A 2 602 750 sind 4,4'-Bis-vinylstilbene sowie aus der CH-A 7000/70 4,4'-Bis-styrylbiphenyle bekannt, welche als optische Aufheller für textile organische Materialien Verwendung finden.

Aufgabe der Erfindung war es, neue als optische Aufheller verwendbare Verbindungen zu schaffen, welche den vorgenannten Verbindungen bezüglich ihrer Aufhelleffekte, Auszieheigenschaften und Lichtechtheiten überlegen sind.

Es wurde nun überraschenderweise gefunden, dass 4-Styryl-4'-vinylbiphenyle diese gewünschten Eigenschaften besitzen und somit die Aufgabe der Erfindung in hervorragender Weise lösen. Diese neuen optischen Aufheller sind auch besonders ausgiebig.

Die neuen 4-Styryl-4'-vinylbiphenyle entsprechen der Formel 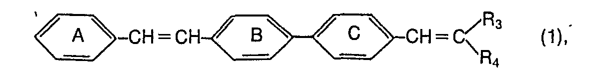 worin die Benzolringe A, B und C gegebenenfalls durch nicht-chromophore Substituenten substituiert sein können und R₃ einen nicht-chromophoren Substituenten 2. Ordnung und R₄ Wasserstoff oder einen nicht-chromophoren Substituenten, der jedoch kein Substituent 2. Ordnung sein kann, bedeuten.

Als nicht-chromophore Substituenten in Verbindungen der Formel (1) kommen vor allem solche in Betracht, die auf dem Gebiet der optischen Aufheller üblich sind. Beispiele hierfür sind: unsubstituiertes oder nicht-chromophor substituiertes Alkyl oder Alkoxy, Alkenyl, Cycloalkyl, Aryl, Aralkyl, Alkoxycarbonyl, gegebenenfalls substituiertes Aminocarbonyl, Cyano, Alkylsulfonyl, Alkoxysulfonyl, gegebenenfalls substituiertes Aminosulfonyl, Acyl, Acylamino, Hydroxy, Aryloxy, Aralkoxy, Alkenyloxy, Aryloxycarbonyl, Aralkyloxycarbonyl, Carboxy, Sulfo, Acyloxy oder Trifluormethyl.

Unter «Aryl» sind vorzugsweise aromatische ein- oder mehrkernige carbocyclische Ringsysteme zu verstehen, z. B. Naphthyl (1) oder Naphthyl (2), vor allem aber Phenyl. In zusammengesetzten Gruppen (z. B. Aryloxy, Aralkyl, Aralkoxy usw.) gilt für Aryl dieselbe Definition.

Nicht-chromophore Substituenten für Alkylgruppen oder Alkoxygruppen sind beispielsweise: Hydroxy, Alkoxy, Alkoxyalkoxy, Hydroxyalkoxy, Halogen, Cyano, Aryl (insbesondere Phenyl), Sulfo, Carboxy, Carbalkoxy, Aminocarbonyl.

Falls Arylgruppen (oder Arylgruppen in zusammengesetzten Resten), insbesondere Phenylreste (oder Phenylreste in zusammengesetzten Resten wie Phenoxy, Phenylalkyl, Phenylsulfonyl usw.) substituiert sind, tragen sie vorzugsweise einen oder zwei Substituenten aus der Gruppe Halogen (insbesondere Chlor), Alkyl und/oder Alkoxy, ferner Sulfo oder Carboxy und deren Derivate, Cyano, Amino, Alkylamino, Dialkylamino und Acyl. Bevorzugte Substituenten sind Chlor, Methyl und Methoxy, von denen auch 3 im Ring vorhanden sein können.

Halogen ist insbesondere Fluor, Chlor oder Brom, vorzugsweise Chlor.

Acyl ist insbesondere Alkylcarbonyl, Alkylsulfonyl, und gegebenenfalls durch Alkyl, Alkoxy oder Halogen substituiertes Phenylsulfonyl.

Alkyl- und Alkoxygruppen haben als solche oder in Alkyl- oder Alkoxygruppen enthaltenden zusammengesetzten Gruppen in der Regel 1 bis 8, insbesondere 1 bis 6, vorzugsweise 1 bis 4 C-Atome. Cycloalkyl weist im Ring vorzugsweise 5 oder 6 C-Atome auf. Alkenylgruppen haben vorzugsweise 2 bis 6, insbesondere 3 oder 4 C-Atome. Alkylreste in Carbonsäureester- oder -amidgruppen oder in Sulfonamidgruppen haben vorzugsweise 1 bis 8 C-Atome.

Nicht-chromophore Substituenten 2. Ordnung sind die in der organischen Chemie bekannten elektronenanziehenden Substituenten, beispielsweise Acyl reste organischer Carbon- oder Sulfonsäuren, Cyano, Trifluormethyl, die Carboxy- und Sulfogruppe und deren funktionelle Derivate wie z. B. deren Salze, Ester und Amide, sowie Derivate von Resten von Phosphor-Sauerstoff-Verbindungen.

Unter Alkoxy sind auch Gruppen der Formel -(OCH₂-CH₂-)ₙ-OR zu verstehen, worin R Wasserstoff oder C₁-C₄-Alkyl und n eine ganze Zahl von 1 bis 6 bedeutet. Ebenso sind als weitere Substituenten solche der Formel -(-CH₂-CH₂-O-)ₙ₋(-C₁-C₄-Alkyl) zu erwähnen, worin n wie vorstehend definiert ist.

Besonders zu erwähnen sind im Rahmen der Verbindungen der Formel (1) solche der Formel 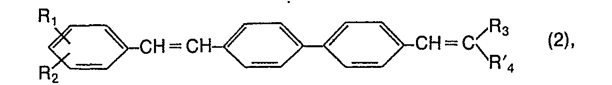 worin R₁ und R₂ jeweils Wasserstoff oder einen nicht-chromophoren Substituenten oder beide zusammen in ortho-Stellung die Ergänzung zu einem anellierten Ring, R₃ einen nicht-chromophoren Substituenten 2. Ordnung und R₄' Wasserstoff oder gegebenenfalls nicht-chromophor substituiertes Alkyl oder Alkenyl bedeuten.

Als nicht-chromophore Substituenten R₁ und R₂ kommen vorzugsweise die weiter oben erwähnten in Betracht. Als anellierte Ringe, die durch R₁ und R₂ gemeinsam ergänzt werden können, kommen vorzugsweise Benzol-, Naphthalin-, Cyclohexen-, oder Cyclopentenringe in Betracht oder die beiden Reste bilden gemeinsam den Methylendioxy-, Äthylendioxy- oder Methylenoxymethylenoxyrest. Bevorzugte nicht-chromophore Substituenten für Alkylgruppen (R₄' ) sind weiter oben erwähnt. Die gleichen Substituenten können auch Alkenylgruppen tragen. Nicht-chromophore Substituenten 2. Ordnung sind ebenfalls unter Formel (1) bereits beispielsweise erläutert worden.

Von praktischem Interesse sind vor allem die Verbindungen der Formel 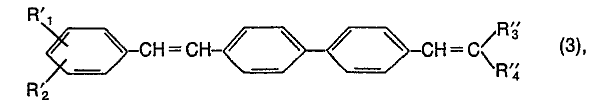 worin Rᵢ' Wasserstoff, Halogen, unsubstituiertes oder nicht-chromophor substituiertes Alkyl, Alkoxy oder Alkylsulfonyl, unsubstituiertes oder nicht-chromophor substituiertes Phenyl, Phenylalkyl oder Phenylsulfonyl, Phenoxy, Phenylalkoxy, *Cyano, eine Gruppe der Formel -COOY₁, -CONY₁Y₂ oder-S0₂NY₁Y₂, worin Y₁ und Y₂ unabhängig voneinander für Wasserstoff, Alkenyl, Propargyl, Cycloalkyl mit 5 oder 6 Ring-C-Atomen, unsubstituiertes oder nicht-chromophor substituiertes Alkyl, Phenyl, Phenylalkyl, oder Y₁ und Y₂ zusammen mit dem Stickstoffatom, an das sie gebunden sind, für einen 5- oder 6-gliedrigen gesättigten heterocyclischen Ring stehen, der gegebenenfalls noch zusätzlich 1 oder 2 Heteroatome als Ringglieder enthalten kann und der gegebenenfalls auch durch Alkylgruppen substituiert sein kann, oder worin Y₁ in der Gruppe -COOY₁ zusätzlich zu den vorstehenden Bedeutungen auch noch für ein salzbildendes Kation stehen kann; oder eine Gruppe der Formel 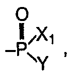 worin X₁ und Y unabhängig voneinander für Halogen, Alkyl, Alkenyl, Phenyl, Phenylalkyl, Hydroxy, Alkoxy, Phenylalkoxy, Cycloalkoxy, Phenoxy, Amino, Mono- oder Dialkylamino, Phenylalkylamino, Acylamino, Phenylamino, Cycloalkylamino, Morpholino, Piperidino oder Pyrrolidino stehen; oder zusammen mit R2 in ortho-Stellung den Rest der Formel -CH=CH-CH=CH-, -O-CH₂-O- oder -O-CH₂CH₂-O-, R₂' Wasserstoff, Halogen, unsubstituiertes oder nicht-chromophor substituiertes Alkyl oder Alkoxy oder zusammen mit R₁' in ortho-Stellung den Rest der Formel -CH=CH-CH=CH-, -O-CH₂-O- oder -O-CH₂- CH₂-O-, R3" Alkylsulfonyl, Phenylsulfonyl, Alkoxysulfonyl, Cyano, Trifluormethyl, Sulfo, eine Gruppe der Formel -COOY₁, -CONY₁Y₂ oder -SO₂NY₁Y₂, worin Y₁ und Y₂ wie oben definiert sind, oder eine Gruppe der Formel 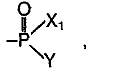 worin X₁ und Y wie oben definiert sind, und R4" Wasserstoff oder unsubstituiertes oder nicht-chromophor substituiertes Alkyl bedeuten.

Beispiele für nicht-chromophore Substituenten für Alkylgruppen (und damit auch für Alkoxy- und Alkylsulfonylgruppen) sowie für Phenylreste und Phenylreste enthaltende Gruppen sind bereits unter Formel (1) angeführt worden.

Falls 5- oder 6-gliedrige gesättigte heterocyclische Ringe (Y₁+Y₂) noch weitere Heteroatome im Ring enthalten, können dies insbesondere 1 oder 2 Stickstoff-, Sauerstoff- und/oder Schwefelatome sein. Als bevorzugte Heterocyclen, die Y₁ und Y₂ gemeinsam mit dem Stickstoffatom bilden können, sind der Piperidin-, Piperazin-, Morpholin-, Thiomorpholin-, Pyrrolidin-, Imidazolidin- und Oxazolidinring zu nennen.

Derartige Heterocyclen können beispielsweise noch mit 1 oder 2 Alkylgruppen substituiert sein, vorzugsweise mit solchen mit 1-4 C-Atomen.

Als salzbildendes Kation Y₁ kommen vorzugsweise Alkalimetallionen (z.B. Na, K), Ammoniumionen oder substituierte Ammoniumionen (Aminsalzionen) in Betracht.

Bevorzugte Verbindungen im Rahmen der Formel (3) entsprechen der Formel 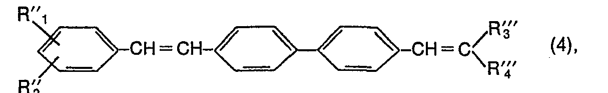 worin
R₁" Wasserstoff, Halogen, C₁-C₄-Alkyl, C₁-C₄-Alkoxy, C₁-C₄-Alkylsulfonyl, Cyano, gegebenenfalls durch Chlor, Methyl oder/und Methoxy substituiertes Phenyl oder Phenylsulfonyl, eine Gruppe der Formel -COOY₁', -CONY₁'Y₂' oder -SO₂-NY₁'Y_{Z} , worin Y₁' für Wasserstoff, C₁-C₈-Alkyl, C₃-C₄-Alkenyl, Cyclohexyl, C_{z}-C₄-Hydroxyalkyl, C₃-C₆-Alkoxyalkyl, einen Rest der Formel -(-CH₂CH₂-0-)₈ (-C₁-C₄-Alkyl), worin n eine ganze Zahl zwischen 1 und 6 ist, C₆-C₉-Phenoxyal- kyl, C₂-C₆-Carboxyalkyl, C₃-C₆-Carbalkoxyalkyl, C₂-C₅-Cyanoalkyl, unsubstituiertes oder durch Chlor, Methyl oder/und Methoxy substituiertes Phenyl oder Benzyl, C₃-C₇-Dialkylaminoalkyl oder Phenäthyl, Y₂ für Wasserstoff, C₁-C₄-Alkyl, C₃-C₄-Alkenyl oder C₂-C₄-Hydroxyalkyl oder Y₁' und Y₂' zusammen mit dem Stickstoffatom, an das sie gebunden sind, für einen 5- oder 6-gliedrigen gesättigten heterocyclischen Ring stehen, der noch ein weiteres Stickstoff- oder Sauerstoffatom als Ringglied enthalten kann und der gegebenenfalls durch 1 oder 2 C₁-C₄-Alkylgruppen substituiert sein kann, oder worin Y₁' in der Gruppe -COOY₁' zusätzlich zu den vorstehenden Bedeutungen noch für ein Alkalimetall- oder Ammoniumion stehen kann; oder eine Gruppe der Formel 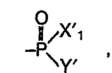 worin X₁' und Y' unabhängig voneinander für C₁-C₄-Alkyl, Benzyl, unsubstituiertes oder durch Chlor, Methyl und/oder Methoxy substituiertes Phenyl stehen,
R2" Wasserstoff, Halogen oder C₁-C₄-Alkyl,
R₃"' Cyano, C₁-C₄-Alkylsulfonyl oder eine Gruppe der Formel -COOY₁', -CONY₁'Y₂ , S0₂NY₁'Y₂ oder 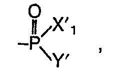 worin Y_{1'} Y₂, X₁' und Y' wie oben definiert sind und
R₄ Wasserstoff oder C₁-C₄-Alkyl bedeuten, sowie der Formel 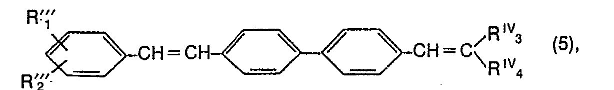 worin
R₁'" Halogen, C₁-C₄-Alkylsulfonyl, Cyano oder eine Gruppe der Formel -COOY₁", worin Y₁" für C₁-C₈-Alkyl, C₃-C₄-Alkenyl, C₂-C₄-Hydroxyalkyl, C₃-C₆-Alkoxyalkyl, einen Rest der Formel (-CH₂CH₂-0-)ₙ-(-C₁-C₄-Alkyl), worin n' eine ganze Zahl zwischen 1 und 4 ist, C₂-C₆-Carboxyalkyl, C₃-C₆-Carbalkoxyalkyl oder C₂-C₅-Cyanoalkyl steht; oder eine Gruppe der Formel 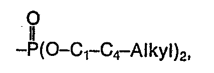
R2 " Wasserstoff oder Halogen,
R3^{IV} C₁-C₄-Alkylsulfonyl, Cyano, eine Gruppe der Formel 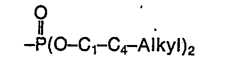 oder eine Gruppe der Formel -COOY₁", worin Y₁" wie oben definiert ist und
R₄^{IV} Wasserstoff oder C₁-C₄-Alkyl bedeuten.

In den Verbindungen der Formeln (1) bis (5) bedeutet R₄, R₄', R₄", R₄^{"'} bzw. R₄^{IV} vorzugsweise Wasserstoff.

Besonders bevorzugt sind die Verbindungen der Formel 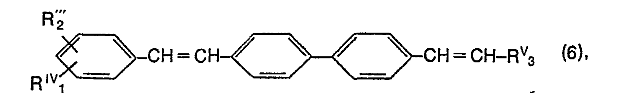 worin R₄^{IV} Cᵢ-C₄-Alkylsulfonyl, Cyano, 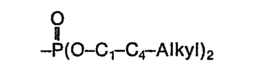 oder -COOY₁"', worin Y"' für C₁-C₄-Alkyl, C₂-C₄-Hydroxyalkyl, C₃-C₆-Alkoxyalkyl, (-CH₂-C0₂-0-)ₙ-(-Cₗ-C₄-Alkyl), worin n' eine ganze Zahl zwischen 1 und 4 ist, C₃-C₆-Carbalkoxyalkyl, C₂-C₆-Carboxyalkyl oder C₂-C₅-Cyanoalkyl steht, R₂"' Wasserstoff oder Halogen und
R₃^{v} C₁-C₄-Akylsulfonyl, Cyano, 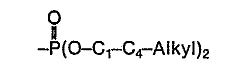 oder-COOY1"', worin Y₁'" wie oben definiert ist, bedeuten.

In praktisch besonders wichtigen Verbindungen der Formel (6) bedeuten R₁^{IV} Cyano oder -COOY₁"', worin Y₁'" für C₁-C₄-Alkyl, C2-C4-Hydroxyalkyl, C₃-C₆-Alkoxyalkyl oder (-CH₂-CH₂-O)ₙ-(-C₁-C₄-Alkyl) steht, worin n' eine ganze Zahl zwischen 1 und 4 ist, R₃^{v} Cyano oder -COOY₁', worin Y₁ wie oben definiert ist, und R₂'" Wasserstoff oder Chlor.

Besonders bevorzugt bedeuten in Formel (6) R₁^{IV} Cyano, R₂'" Wasserstoff und R₃^{v} COOY₁^{IV}für C₁-C₄-Alkyl, C₂-C₄-Hydroxyalkyl, C3-C6-Afkoxyal- kyl oder (-UCH₂-CH₂-O-)ₙ-(-C₁-C₄-Alkyl) steht, worin n' eine ganze Zahl zwischen 1 und 4 ist, wobei aber auch R₃^{v} vorzugsweise für CN steht.

Die erfindungsgemässen 4-Styryl-4'-vinylbiphe- nyle der Formel (1) und somit auch der untergeordneten Formeln (2) bis (6) können beispielsweise nach einem neuen Verfahren hergestellt werden, das darin besteht, dass man einen Biphenyl-4,4'-dialdehyd der Formel 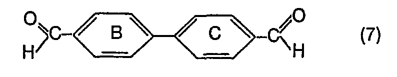 mit einer Verbindung der Formel 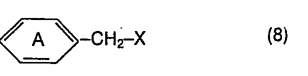 zu einem Aldehyd der Formel 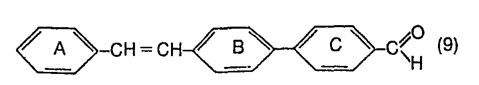 umsetzt und diesen Aldehyd dann weiter mit einer Verbindung der Formel 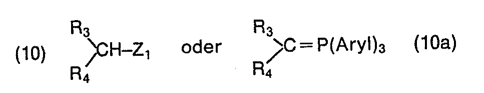 zu einer in Formel (1) definierten Verbindung umsetzt, wobei A, B, C, R₃ und R₄ wie in Formel (1) definiert sind und wobei X und Z₁ gleich oder verschieden sind und unabhängig voneinander Wasserstoff oder einen Rest der Formel -COOZ, worin Z für Wasserstoff oder Alkyl steht, 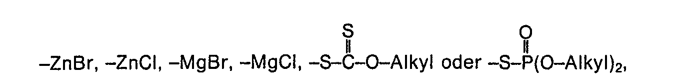 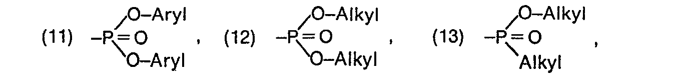 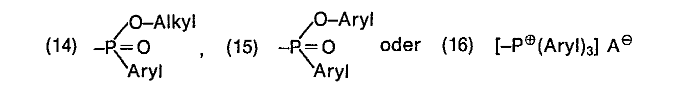 bedeuten, worin A⁹ für ein einwertiges farbloses Anion steht, und dass man gegebenenfalls eine so erhaltene, in Formel (1) definierte Verbindung durch übliche Methoden in eine andere von Formel (1) umfasste Verbindung umwandelt.

Als farbloses Anion A° kann jedes beliebige Anion fungieren; es hat keinen Einfluss auf die Funktionsfähigkeit des erfindungsgemässen Verfahrens. Bevorzugte Anionen A⊖ sind Halogenidionen, z.B. Chlorid oder Bromid, Alkylsulfat-oder Arylsulfonationen, z.B. Phenylsulfonat-, Tolylsulfonat-, und Chlorphenylsulfonationen. Aber auch andere übliche Anionen können verwendet werden. Die bevorzugten Bedeutungen für «Aryl» in Formel (10a) und den Formeln (11), (14), (15) und (16) sind dieselben wie eingangs aufgezählt.

Nach dem erfindungsgemässen Verfahren erhaltene Verbindungen der Formel (1) können durch an sich bekannte Methoden in andere Verbindungen der Formel (1) umgewandelt werden, beispielsweise durch Veresterung, Hydrolyse, Umesterung, Oxidation, Reduktion, Halogenierung, Amidierung usw. Beispielsweise kann eine Carbonsäureestergruppe R_{1'} R₂ und/oder R₃ zur entsprechenden Säure hydrolysiert, letztere in das Säurechlorid übergeführt und dieses mit einem Alkohol zu einer anderen Carbonsäureestergruppe umgesetzt werden.

Bevorzugt wird das oben beschriebene erfindungsgemässe Verfahren in der Weise durchgeführt, dass man als Verbindungen der Formel (8) bzw. (10) solche einsetzt, in denen X bzw. Zᵢ unabhängig voneinander jeweils eine Gruppe der Formel (11), (12), (13), (14) oder (15), vorzugsweise eine Gruppe der Formel (12) bedeuten. In den Formeln (11)-(16) haben Alkylgruppen vorzugsweise 1 bis 6, insbesondere 1 bis 4 C-Atome. Als Arylreste kommen insbesondere ein unsubstituierter Phenylrest oder ein mit Chlor oder Cᵢ-C₄-Alkyl substituierter Phenylrest in Frage.

Alternativ können die Verbindungen der Formel (1) auch hergestellt werden, indem man einen Biphenyl-4,4'-dialdehyd der Formel 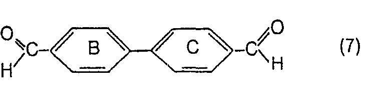 mit einer Verbindung der Formel 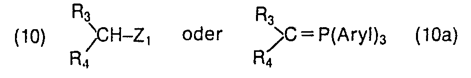 zu einem Aldehyd der Formel 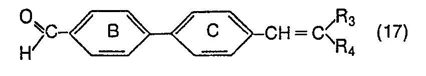 umsetzt und diesen Aldehyd dann weiter mit einer Verbindung der Formel 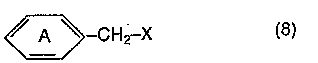 zu einer in Formel (1) definierten Verbindung umsetzt, wobei in den obigen Formeln A, B, C, R₃, R₄, X und Z₁ die in den Formeln (1), (8) und (10) weiter oben angegebenen Bedeutungen haben.

Auch in diesem Verfahren werden vorzugsweise solche Verbindungen der Formeln (8) und (10) eingesetzt, in denen X bzw. Z₁ jeweils eine der Gruppen der Formeln (11) bis (15), vorzugsweise eine Gruppe der Formel (12) bedeuten.

Die Umsetzung einer Verbindung der Formel (7), vorzugsweise von Biphenyl-4,4'-dialdehyd selbst mit einer Verbindung der Formel (8) bzw. mit einer Verbindung der Formel (10) oder (10a) (erste Stufe) wird vorzugsweise in Gegenwart eines alkalischen Kondensationsmittels, das als Protonenakzeptor dient, durchgeführt. Als derartige Kondensationsmittel kommen anorganische oder organische Basen in Betracht, z.B. Hydroxide, Hydride, Alkoxide und Amide der Alkali- oder Erdalkalimetalle, monomere oder polymere stark basische Amine, quaternäre Ammoniumhydroxyde und Harzaustauscher der OH-Reihe in Frage. Von besonderer praktischer Bedeutung sind Natrium- und Kaliumhydroxid, Natriummethylat und Natriumäthylat. Auch ein Gemisch verschiedener Basen kann verwendet werden. Die Menge an einzusetzendem Kondensationsmittel bewegt sich in weiten Grenzen. Vorteilhaft verwendet man die äquivalente Menge, es kann jedoch auch ein Überschuss verwendet werden. Auch die zweite Stufe [Umsetzung der Monoaldehyde der Formel (9) oder (17) mit einer Verbindung der Formel (10) oder (10a) bzw. der Formel (8)] wird vorzugsweise in Gegenwart der gleichen Kondensationsmittel durchgeführt wie für die erste Stufe beschrieben.

Das erfindungsgemässe Verfahren wird zweckmässig in einem unter den Reaktionsbedingungen inerten Lösungsmittel durchgeführt. Als solche Lösungsmittel kommen apolare und dipolare aprotische und protische Lösungsmittel wie z.B. Hexan, Octan, Cyclohexan, Toluol, Xylol, Chlorbenzol usw.; Formamid, Dimethylformamid, N-Methylpyrrolidon, Acetonitril, Dimethylsulfoxyd usw.; Methanol, Äthanol, Isopropanol, Hexanol, usw. in Betracht. Auch in Wasser oder in Wasser enthaltenden Gemischen in Gegenwart oder Abwesenheit von Phasentransferkatalysatoren lässt sich das Verfahren der Erfindung durchführen.

Die erste Stufe wird vorzugsweise in einem Lösungsmittel, in welchem die entstehenden Monoaldehyde der Formeln (9) bzw. (17) wenig löslich sind, wie z.B. in Methanol, Äthanol, Hexan oder Toluol, durchgeführt. Die entstehenden Aldehyde fallen während der Umsetzung zusammen mit kleinen Anteilen der entsprechenden symmetrischen Verbindungen aus und können durch Filtration isoliert oder vorzugsweise ohne Isolierung weiter umgesetzt werden. Werden die erhaltenen Zwischenprodukte isoliert, dann werden sie vorzugsweise ohne Reinigung weiter eingesetzt.

Die Umsetzung der Monoaldehyde der Formeln (9) bzw. (17) mit einer Verbindung der Formel (10) oder (10a) bzw. der Formel (8) (zweite Stufe) wird vorzugsweise in einem Lösungsmittel, in welchem die Monoaldehyde teilweise oder vollständig löslich sind, durchgeführt. Als solche Lösungsmittel kommen vor allem aprotische dipolare Lösungsmittel wie z. B. Dimethyl- und Diäthylformamid und Dimethylsulfoxid in Frage.

Die Reaktionstemperatur bewegt sich in Abhängigkeit vom gewählten Lösungsmittel in weiten Grenzen und kann leicht durch Vorversuche ermittelt werden. Die erste Stufe wird zweckmässig bei Temperaturen zwischen 0 °C und 50 °C, vorzugsweise zwischen 20 °C und 30 °C durchgeführt. Für die zweite Stufe kommen vor allem Temperaturen zwischen 20 °C und 100 °C, vorzugsweise zwischen 30 °C und 50 °C in Betracht.

Die in den erfindungsgemässen Verfahren als Ausgangsprodukte eingesetzten Verbindungen der Formeln (8), (10) und (10a) sind bekannt oder können in Analogie zu bekannten Verfahren leicht hergestellt werden. Siehe dazu DE-A 1 921 466, GB-B 920 988 und 929 436 und DE-A 2 602 750. Die Ausgangsverbindungen der Formel (7), insbesondere der unsubstituierte Biphenyl-4,4'-dialdehyd, sind ebenfalls bekannt. Substituierte Verbindungen der Formel (7) können ähnlich wie der unsubstituierte Dialdehyd erhalten werden.

Die im erstgenannten erfindungsgemässen Verfahren als Zwischenprodukte anfallenden Monoaldehyde der Formel (9) sind neu.

Von besonderem Interesse unter den Verbindungen der Formel (9) sind solche der Formel 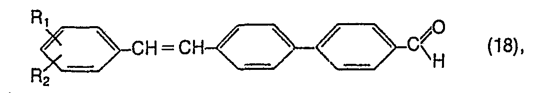 worin R₁ und R₂ jeweils Wasserstoff oder einen nicht-chromophoren Substituenten oder beide zusammen in ortho-Stellung die Ergänzung zu einem anellierten Ring bedeuten, wobei Rᵢ vorzugsweise Wasserstoff, Halogen, unsubstituiertes oder nicht-chromophor substituiertes Alkyl, Alkoxy oder Alkylsulfonyl, unsubstituiertes oder nicht-chromophor substituiertes Phenyl, Phenylalkyl oder Phenylsulfonyl, Phenoxy, Phenylalkoxy, Cyano, eine Gruppe der Formel -COOY₁, -CONY₁Y₂ oder -SO₂NY₁Y₂, worin Y₁ und Y₂ unabhängig voneinander für Wasserstoff, Alkenyl, Propargyl, Cycloalkyl mit 5 oder 6 Ring-C-Atomen, unsubstituiertes oder nicht-chromophor substituiertes Alkyl, Phenyl, Phenylalkyl, oder Y₁ und Y₂ zusammen mit dem Stickstoffatom, an das sie gebunden sind, für einen 5- oder 6-gliedrigen gesättigten heterocyclischen Ring stehen, der gegebenenfalls noch zusätzlich 1 oder 2 Heteroatome als Ringglieder enthalten kann und der gegebenenfalls auch durch Alkylgruppen substituiert sein kann, oder worin Y₁ in der Gruppe-COOY₁ zusätzlich zu den vorstehenden Bedeutungen auch noch für ein salzbildendes Kation stehen kann; oder eine Gruppe der Formel 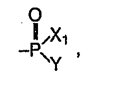 worin Xᵢ und Y unabhängig voneinander für Halogen, Alkyl, Alkenyl, Phenyl, Phenylalkyl, Hydroxy, Alkoxy, Phenylalkoxy, Cycloalkoxy, Phenoxy, Amino, Mono- oder Dialkylamino, Phenylalkylamino, Acylamino, Phenylamino, Cycloalkylamino, Morpholino, Piperidino oder Pyrrolidino stehen; oder zusammen mit R₂ in ortho-Stellung den Rest der Formel -CH=CH-CH=CH-, -O-CH_{z}-O- oder -O-CH₂-CH₂-O- und R₂ vorzugsweise Wasserstoff, Halogen, unsubstituiertes oder nicht-chromophor substituiertes Alkyl oder Alkoxy oder zusammen mit Rᵢ in ortho-Stellung den Rest der Formel -CH=CH-CH=CH-, -O-CH2-O- oder -OCH₂ CH₂-O- bedeuten.

Hervorzuheben sind auch die Verbindungen der Formel 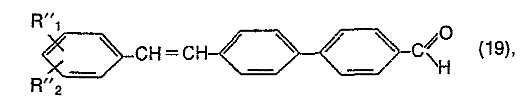 worin
R₁" Wasserstoff, Halogen, C₁-C₄-Alkyl, C₁-C₄-Alkoxy, C₁-C₄-Alkylsulfonyl, Cyano, gegebenenfalls durch Chlor, Methyl oder/und Methoxy substituiertes Phenyl oder Phenylsulfonyl, eine Gruppe der Formel -COOY₁', -CONY₁'Y₂' oder -SO₂-NY₁'Y₂ , worin Y₁' für Wasserstoff, C₁-Cₐ-Alkyl, C₃-C₄-Alkenyl, Cyclohexyl, C₂-C₄-Hydroxyalkyl, C₃-C₆-Alkoxyalkyl, 'einen Rest der Formel -(-CH₂CH₂-O-)n-(-C₁-C₄-Alkyl), worin n eine ganze Zahl zwischen 1 und 6 ist, C₆-C₉-Phenoxyal- kyl, C₂-C₆-Carboxyalkyl, C₃-C₆-Carbalkoxyalkyl, C₂-C₅-Cyanoalkyl, unsubstituiertes oder durch Chlor, Methyl oder/und Methoxy substituiertes Phenyl oder Benzyl, C₃-C₇-Dialkylaminoalkyl oder Phenäthyl, Y2 für Wasserstoff, C₁-C₄-Alkyl, C3-C4-Alkenyl oder C₂-C₄-Hydroxyalkyl oder Y₁' und Y₂' zusammen mit dem Stickstoffatom, an das sie gebunden sind, für einen 5- oder 6-gliedrigen gesättigten heterocyclischen Ring stehen, der noch ein weiteres Stickstoff- oder Sauerstoffatom als Ringglied enthalten kann und der gegebenenfalls durch 1 oder 2 C₁-C₄-Alkylgruppen substituiert sein kann, oder worin Y₁' in der Gruppe -COOY₁' zusätzlich zu den vorstehenden Bedeutungen noch für ein Alkalimetall- oder Ammoniumion stehen kann; oder eine Gruppe der Formel 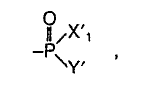 worin X,' und Y' unabhängig voneinander für C₁-C₄-Alkyl, Benzyl, unsubstituiertes oder durch Chlor, Methyl oder/und Methoxy substituiertes Phenyl stehen, und R2" Wasserstoff, Halogen oder Cᵢ-C₄-Alkyl bedeuten, wobei R₁" vorzugsweise Halogen, C₁-C₄-Alkylsulfonyl, Cyano oder eine Gruppe der Formel -COOY₁", worin Y₁" für C₁-C₈-Alkyl, C₃-C₄-Alkenyl, C₂-C₄-Hydroxyalkyl, C₃-C₆-Alkoxyalkyl, einen Rest der Formel (-CH₂CH₂-O-)ₙ-(-C₁-C₄-Alkyl), worin n' eine ganze Zahl zwischen 1 und 4 ist, C₂-C₆-Carboxyalkyl, C₃-C₆-Carbalkoxyalkyl oder C_{z}-C₅-Cyanoalkyl steht; oder eine Gruppe der Formel 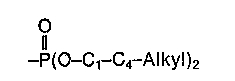
und R2" vorzugsweise Wasserstoff oder Halogen bedeuten.

Bevorzugte Zwischenprodukte entsprechen der Formel 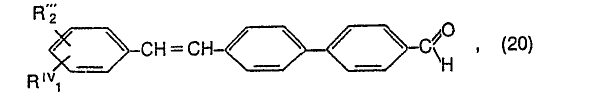 worin R₁^{IV} C₁-C₄-Alkylsulfonyl, Cyano, 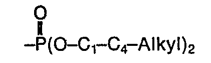 oder -COOY₁"', worin Y₁"' für
C₁-C₄-Alkyl, C₂-C₄-Hydroxyalkyl,
C₃-C₆-Alkoxyalkyl,
(-CH₂-CH₂-0-)ₙ-(-C₁-C₄-Alkyl),

worin n' eine ganze Zahl zwischen 1 und 4 ist, C₃-C₆-Carbalkoxyalkyl, C₂-C₆-Carboxyalkyl oder C₂-C₅-Cyanoalkyl steht, und R₂"' Wasserstoff oder Halogen bedeuten, wobei vorzugsweise R₁^{IV} Cyano oder -COOY₁"', worin Y₁"' für
Cᵢ-C₄-Alkyl, C_{z}-C₄-Hydroxyalkyl,
C₃-C₆-Alkoxyalkyl,
(-CH₂-CH₂-O-)ₙ-(-C₁-C₄-Alkyl)

steht, worin n' eine ganze Zahl zwischen 1 und 4 ist, und R₂"' Wasserstoff oder Chlor bedeuten.

Besonders bevorzugt sind solche Verbindungen der Formel (20), in denen R₁^{IV} für Cyano und R₂ für Wasserstoff steht.

Die Zwischenprodukte der Formel (9) werden vorzugsweise dadurch hergestellt, dass man ein Biphenyl-4,4'-dialdehyd der Formel 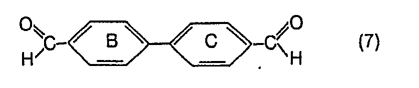 mit einer Verbinduna der Formel 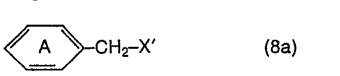 umsetzt, wobei in obigen Formeln A, B und C wie in Formel (1) definiert ist und X' einen Rest der Formel 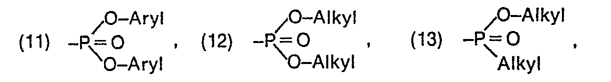 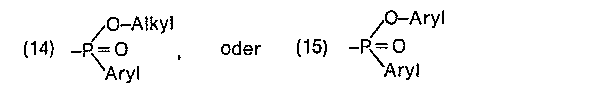 bedeutet.

Dieses Verfahren entspricht der ersten Stufe des erstgenannten Verfahrens für die Herstellung von 4-Styryl-4'-vinylbiphenylen der Formel (1). Der entstehende Monoaldehyd der Formel (9) bzw. der untergeordneten Formeln (18)-(20) kann in üblicher Weise aus dem Reaktionsgemisch isoliert und gereinigt werden, beispielsweise durch gegebenenfalls mehrmalige Umkristallisation, gegebenenfalls unter Zusatz von Aktivkohle oder Bleicherde. Die Reinigung kann gegebenenfalls auch durch Säulenchromatographie erfolgen.

Bei der Herstellung der Zwischenprodukte der Formel (9) können dieselben bevorzugten Reaktionsbedingungen (Kondensationsmittel, Lösungsmittel, Temperatur usw.) angewendet werden, wie sie für die erste Stufe des erfindungsgemässen Verfahrens zur Herstellung von Verbindungen der Formel (1) weiter oben beschrieben wurden.

Die bei der zweiten Variante des Verfahrens zur Herstellung von Verbindungen der Formel (1) (Umsetzung eines Biphenyl-4,4'-dialdehyds der Formel (7) zuerst mit einer Verbindung der Formel (10) oder (10a) und anschliessend mit einer Verbindung der Formel (8)) als Zwischenprodukte anfallenden Monoaldehyde der Formel (17) sind ebenfalls neu. Bevorzugte Monoaldehyde der Formel (17) entsprechen der Formel 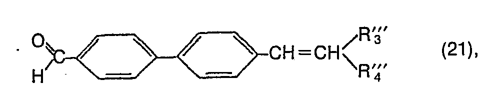 worin R₃"' und R4'" wie in Formel (4) definiert sind. Bevorzugt haben R₃"' und R4 die Bedeutungen von R₃'^{v} und R₄^{IV}, wie sie in Formel (5) definiert sind. Insbesondere steht in besonders interessanten Zwischenprodukten der Formel (21) R₄'" für Wasserstoff und R₃"' für Cyano oder -COOY₁^{IV}, worin Y₁^{IV}C₁-C₄-Alkyl, C_{z}-C₄-Hydroxyalkyl, C₃-C₆-Alkoxyalkyl, -(-CH₂-CH₂-O-)ₙ-(-C₁-C₄-Alkyl), worin n' eine ganze Zahl zwischen 1 und 4 ist, C₃-C₆-Carbalkoxyalkyl oder C₂-C_{S}-Cyanoalkyl bedeutet, vorzugsweise aber steht R₃'" für Cyano.

Die Herstellung der Zwischenprodukte der Formel (17) erfolgt vorzugsweise durch Umsetzung einer Verbindung der Formel (7) mit einer Verbindung der Formel (10) oder (10a), wobei in der Formel (10) der Substituent Zᵢ für eine der Gruppen der Formeln (11) bis (15) steht.

Dieses Verfahren entspricht der ersten Stufe des zweitgenannten Verfahrens für die Herstellung von 4-Styryl-4'-vinylbiphenylen der Formel (1). Der entstehende Monoaldehyd der Formel (17) kann in üblicher Weise aus dem Reaktionsgemisch isoliert und gereinigt werden, beispielsweise durch gegebenenfalls mehrmalige Umkristallisation, gegebenenfalls unter Zusatz von Aktivkohle oder Bleicherde. Die Reinigung kann gegebenenfalls auch durch Säulenchromatographie erfolgen.

Bei der Herstellung der Zwischenprodukte der Formel (17) können dieselben bevorzugten Reaktionsbedingungen (Kondensationsmittel, Lösungsmittel, Temperatur usw.) angewendet werden, wie sie für die erste Stufe des erfindungsgemässen Verfahrens zur Herstellung von Verbindungen der Formel (1) weiter oben beschrieben wurden.

Die erfindungsgemässen 4-Styryl-4'-vinylbiphe- nyle der Formel (1) werden zum optischen Aufhellen der verschiedensten synthetischen, halbsynthetischen oder natürlichen hochmolekularen organischen Materialien verwendet. Diese Verwendung ist ebenfalls Gegenstand der vorliegenden Erfindung.

Als aufzuhellende Materialien seien beispielsweise, ohne dass durch die nachfolgende Übersicht irgendeine Beschränkung hierauf ausgedrückt werden soll, die folgenden Gruppen von organischen Materialien, soweit eine optische Aufhellung derselben in Betracht kommt, genannt:
I. Synthetische organische hochmolekulare Materialien:
   a) Polymerisationsprodukte auf Basis mindestens eine polymerisierbare Kohlenstoff-Kohlenstoff-Doppelbindung enthaltender organischer Verbindungen, d. h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung reaktionsfähiger Gruppen erhaltene Produkte, beispielsweise Polymerisate auf Basis von α,β-ungesättigten Carbonsäuren oder Derivaten solcher Carbonsäuren, insbesondere von Acrylverbindungen (wie z.B. Acrylestern, Acrylsäure, Acrylnitril, Acrylamiden und deren Derivaten oder deren Methacryl-Analoga), von Olefin-Kohlenwasserstoffen (wie z. B. Äthylen, Propylen, Styrole oder Diene, ferner sogenannte ABS-Polymerisate), Polymerisate auf Basis von Vinyl- und Vinyliden-Verbindungen (wie z.B. Vinylchlorid, Vinylalkohol, Vinylidenchlorid),
   b) Polymerisationsprodukte, die durch Ringöffnung erhältlich sind, z.B. Polyamide vom Polycaprolactam-Typ, ferner Polymere, die sowohl über Polyaddition als auch Polykondensation erhältlich sind, wie Polyäther oder Polyacetale,
   c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z.B. Äthylenglykolterephthalsäure-Polyester) oder ungesättigte (z.B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z.B. Alkydharze) Polyester, Polyamide (z.B. Hexamethylendiamin-adipat), Maleinatharze, Melaminharze, deren Vorkondensate und Analoga, Polycarbonate, Silikone,
   d) Polyadditionsprodukte wie Polyurethane (vernetzt und unvernetzt), Epoxidharze.
11. Halbsynthetische organische Materialien, z.B. Celluloseester verschiedener Veresterungsgrade (sogenanntes 2¹/₂-Acetat, Triacetat) oder Celluloseäther, regenerierte Cellulose (Viskose, Kupferammoniak-Cellulose) oder deren Nachbehandlungsprodukte, Casein-Kunststoffe.
111. Natürliche organische Materialien animalischen oder vegetabilischen Ursprungs, beispielsweise auf Basis von Cellulose oder Proteinen wie Baumwolle, Wolle, Leinen, Seide, natürliche Lackharze, Stärke, Casein.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilde vorliegen, d. h. beispielsweise als vorwiegend dreidimensional ausgedehnte Körper wie Platten, Profile, Spritzgussformlinge, verschiedenartige Werkstücke, Schnitzel, Granulate oder Schaumstoffe, ferner als vorwiegend zweidimensional ausgebildete Körper wie Filme, Folien, Lakke, Überzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z.B. als Pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflokkungs-Gebilde oder als textile Gewebe oder textile Verbundstoffe, Gewirke sowie als Papiere, Pappen oder Papiermassen vorliegen.

Den erfindungsgemäss anzuwendenden Verbindungen der Formel (1) kommt vor allem Bedeutung für die Behandlung von textilen organischen Materialien, insbesondere textilen Geweben, zu. Sofern Fasern, welche als Stapelfasern oder Endlosfäden, in Form von Strangen, Geweben, Gewirken, Vliesen, beflockten Substraten oder Verbundstoffen vorliegen können, erfindungsgemäss optisch aufzuhellen sind, geschieht dies mit Vorteil in wässerigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

Bei der Applikation kann man in neutralem, alkalischem oder saurem Bade arbeiten. Die Behandlung wird üblicherweise bei Temperaturen von etwa 20 bis 140 °C, beispielsweise bei Siedetemperatur des Bades oder in deren Nähe (etwa 90 °C), durchgeführt. Für die erfindungsgemässe Veredlung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die optischen Aufheller gemäss vorliegender Erfindung können ferner den Materialien vor oder während deren Verformung zugesetzt bzw. einverleibt werden. So kann man sie beispielsweise bei der Herstellung von Filmen, Folien (z.B. Einwalzen in Polyvinylchlorid in der Hitze) oder Formkörpern der Pressmasse oder Spritzgussmasse beifügen.

Sofern die Formgebung voll- oder halbsynthetischer organischer Materialien durch Spinnverfahren bzw. über Spinnmassen erfolgt, können die optischen Aufheller nach folgenden Verfahren appliziert werden:
- Zugabe zu den Ausgangssubstanzen (z. B. Monomeren) oder Zwischenprodukten (z.B. Vorkondensaten, Praepolymeren), d. h. vor oder während der Polymerisation, Polykondensation oder Polyaddition,
- Aufpudern auf Polymerisatschnitzel oder Granulate für Spinnmassen,
- Badfärbung von Polymerisatschnitzeln oder Granulaten für Spinnmassen,
- Dosierte Zugabe zu Spinnschmelzen oder Spinnlösungen,
- Applikation auf Spinnkabel vor dem Verstrekken.

Die erfindungsgemässen optischen Aufheller können beispielsweise auch in folgenden Anwendungsformen eingesetzt werden:
a) Mischungen mit Farbstoffen (Nuancierung) oder Pigmenten (Farb- oder insbesondere z.B. Weisspigmenten) oder als Zusatz zu Färbebädern, Druck-, Ätz- oder Reservepasten, ferner auch zur Nachbehandlung von Färbungen, Drukken oder Ätzdrucken,
b) in Mischungen mit sogenannten «Carriern_{"}, Netzmitteln, Weichmachern, Quellmitteln. Antioxydantien, Lichtschutzmitteln, Hitzestabilisatoren, chemischen Bleichmitteln (Chlorit-Bleiche, Bleichbäder-Zusätze),
c) in Mischung mit Vernetzern, Appreturmitteln (z. B. Stärke oder synthetischen Appreturen) sowie in Kombination mit den verschiedensten Textilveredlungsverfahren, insbesondere Kunstharzausrüstungen (z.B. Knitterfest-Ausrüstungen wie «wash-and-wear», «permanent-press», «noniron»), ferner Flammfest-, Weichgriff-, Schmutzablöse («anti-soiling»)- oder Antistatisch-Ausrüstungen oder antimikrobiellen Ausrüstungen,
d) Einarbeiten der optischen Aufhellmittel in polymere Trägermaterialien (Polymerisations-, Polykondensations- oder Polyadditionsprodukte) in gelöster oder dispergierter Form für Anwendung z.B. in Beschichtungs-, Imprägnier- oder Bindemitteln (Lösungen, Dispersionen, Emulsionen) für Textilien, Vliese, Papier, Leder,
e) als Zusätze zu den verschiedensten industriellen Produkten, um dieselben marktfähiger zu machen (z. B. Aspektverbesserung von Seifen, Waschmitteln, Pigmenten),
f) in Spinnbadpräparationen, d. h. als Zusätze zu Spinnbädern, wie sie zur Gleitfähigkeitsverbesserung für die Weiterverarbeitung von SyntheseFasern verwendet werden, oder aus einem speziellen Bad vor der Verstreckung der Faser,
g) in Mitteln zum optischen Aufhellen von hochmolekularen organischen Materialien der vorstehend angegebenen Zusammensetzungen, welche Mittel gegebenenfalls übliche Formulierungszusätze und/oder gegebenenfalls weitere optische Aufheller aus anderen Aufhellerklassen enthalten,
h) in Kombination mit anderen, optisch aufhellend wirkenden Substanzen; ferner als Zusätze zu sogenannten «master batches»,
i) als Scintillatoren, für verschiedene Zwecke photographischer Art, wie z. B. für elektrophotographische Reproduktion und Supersensibilisierung,
j) je nach Substitution als Laser-Farbstoffe.

Mittel, die die erfindungsgemässen optischen Aufheller der Formel (1) enthalten, sind ebenfalls Gegenstand der Erfindung.

Als übliche Formulierungszusätze, die in solchen Mitteln enthalten sein können, kommen z. B. verschiedenste Hilfs- und Coupiermittel, wie z.B. wasserfreies Natriumsulfat, Natriumsulfat-decahydrat, Natriumchlorid, Natriumcarbonat, Alkalimetallphosphate, wie Natrium- oder Kaliumorthophosphat, Natrium- oder Kaliumpyrophosphat und Natrium- oder Kaliumtripolyphosphate oder Alkalimetallsilicate in Betracht. Aber auch wässrige Formulierungen fallen unter die erfindungsgemässen Mittel, z. B. auch die Applikationslösungen, mit denen Textilfasern optisch aufgehellt werden und die üblichen Zusätze enthalten.

Besonders bevorzugt im Rahmen der erfindungsgemässen Mittel sind solche, die neben einem auf dem zu behandelnden Substrat eine rötliche Nuance hervorrufenden erfindungsgemässen optischen Aufheller der Formeln (1) bis (6) zusätzlich noch einen optischen Aufheller enthalten, der auf dem zu behandelnden Substrat eine grünliche bis bläuliche Nuance hervorruft.

Derartige Kombinationen haben den Vorteil, dass dadurch auf Textilfasern, vor allem auf Polyesterfasern ein besonders schöner neutraler Weisston von hoher Brillanz erzielt werden kann.

Sehr vorteilhaft sind daher Mittel, die einen oder mehrere optische Aufheller der Formeln (1) bis (6) und zusätzlich einen oder mehrere optische Aufheller aus der Klasse der Bis-styrylbenzole, Benzoxazolylstilbene,
4,4'-Divinylstilbene, Naphthalimide,
4,4'-Bis-styrylbiphenyle,
4,4'-Bis-triazolylstifbene,
Bis-benzoxazolyl-thiophene,
Naphthotriazol-2-yl-stilbene,

(bekannt aus den deutschen Offenlegungsschriften 2 539 537 und 2 539 461) oder der Cumarine, z.B. der Triazolylcumarine (bekannt aus den Schweizer Patentschriften 566 359 und 592 189), enthalten, insbesondere solche, die als Aufheller-Aktivsubstanz 10-99%, insbesondere 30-70%, eines erfindungsgemässen optischen Aufhellers der Formeln (1) bis (6) und 90-1%, insbesondere 70-30% eines optischen Aufhellers aus den oben genannten Klassen enthalten. Derartige Kombinationen von optischen Aufhellern müssen keine weiteren Zusätze enthalten, können also auch als reine Aufhellermischungen vorliegen.

Besonders bevorzugt werden mit den erfindungsgemässen optischen Aufhellern Substrate aus Polyester aufgehellt, insbesondere Textilmaterialien aus Polyester.

Wird das Aufhellverfahren mit Textilbehandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, dass der gewünschte Aufhelleffekt erreicht wird.

In gewissen Fällen werden die Aufheller durch eine Nachbehandlung zur vollen Wirkung gebracht. Diese kann beispielsweise eine chemische (z.B. Säure-Behandlung), eine thermische oder eine kombinierte chemisch/thermische Behandlung darstellen. So verfährt man beispielsweise bei einer optischen Aufhellung einer Reihe von Fasersubstraten, z.B. von Polyesterfasern, mit den erfindungsgemässen Aufhellern zweckmässig in der Weise, dass man diese Fasern mit den wässerigen Dispersionen (gegebenenfalls auch Lösungen) der Aufhellmittel bei Temperaturen unter 75 °C, z.B. bei Raumtemperatur, imprägniert und einer trockenen Wärmebehandlung bei Temperaturen über 100 °C unterwirft, wobei es sich im allgemeinen empfiehlt, das Fasermaterial vorher noch bei mässig erhöhter Temperatur, z. B. bei mindestens 60 °C bis etwa 130 °C zu trocknen. Die Wärmebehandlung in trockenem Zustande erfolgt dann vorteilhaft bei Temperaturen zwischen 120 und 225 °C, beispielsweise durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt oder in einem einzigen Arbeitsgang zusammengelegt werden.

Die Menge der erfindungsgemäss zu verwendenden optischen Aufheller, bezogen auf das optisch aufzuhellende Material, kann in weiten Grenzen schwanken. Schon mit sehr geringen Mengen, in gewissen Fällen z.B. solchen von 0,001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,01 und 0,5 Gewichtsprozent von Interesse.

Besonders bevorzugte Anwendungsgebiete für die erfindungsgemässen Verbindungen sind die folgenden:
Optisches Aufhellen von Polyester, und zwar sowohl von Polyesterfasern und -gewebe nach dem Auszieh- oder Foulardthermverfahren, als auch von Polyesterspinnmassen. Mischgewebe aus Polyester und Baumwolle oderWolle werden ebenfalls sehr vorteilhaft mit Hilfe der erfindungsgemässen Verbindungen aufgehellt. Beispiele für weitere Substrate, die vorteilhaft mit Hilfe der Verbindungen der Formel (1) aufgehellt werden können, sind: Polyamidfasergewebe, Celluloseacetatgewebe sowie Polystyrol- und Polyvinylchloridmassen. Besonders bevorzugt ist jedoch die Verwendung zum optischen Aufhellen von Polyesterfasern nach dem Auszieh- oder Foulardthermverfahren.

Die nachfolgenden Beispiele erläutern die Herstellung der erfindungsgemässen Verbindungen sowie deren Verwendung weiter. In diesen Beispielen, ebenso wie in der übrigen Beschreibung, sind Prozent- und Teilangaben immer auf das Gewicht bezogen, sofern nichts anderes vermerkt ist. Schmelz- und Siedepunkte sind, wenn nicht anders angegeben, unkorrigiert.

### Beispiel 1:

a) 21 g Biphenyl-4,4'-dialdehyd werden in 200 ml wasserfreiem Methanol suspendiert und bei 20 bis 25 °C unter Rühren und Stickstoff innerhalb 15 Minuten mit 36 g einer 30%igen methanolischen Natriummethylat-Lösung versetzt. Es entsteht eine fast klare Lösung, die bei 20 bis 25 °C unter Rühren und Stickstoff innerhatB 10 Minuten mit einer Lösung von 25,3 g des Phosphonates der Formel 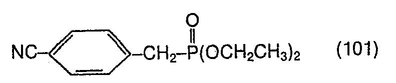 in 50 ml wasserfreiem Methanol versetzt wird, wobei das Reaktionsprodukt sofort kristallin ausfällt. Der entstandene dicke kristalline Reaktionsbrei wird nun 20 Stunden bei 20 bis 25 °C unter Stickstoff weitergerührt, dann abgenutscht, mit etwa 50 ml wasserfreiem Methanol gewaschen und unter Vakuum bei 80 °C bis zum konstanten Gewicht getrocknet. Man erhält 29 g (etwa 93,7% der Theorie) des Aldehyds der Formel 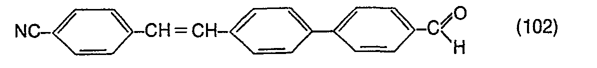 in Form eines gelben kristallinen Pulvers mit einem Schmelzpunkt von 192 bis 196 °C.

Nach zweimaligem Umkristallisieren aus Chlorbenzol erhält man 19 g der Verbindung der Formel (102) in Form von gelben Nadeln mit einem Schmelzpunkt von 197 bis 200 °C.

Das als Ausgangsmaterial verwendete Phosphonat der Formel (101) wird gemäss Beispiel 1 der deutschen Offenlegungsschrift 1 921 466 hergestellt.

b) 15,5 g des 4-(4-Cyanostyryl)-biphenyl-4'-aldehyds der Formel (102) und 11,2 g Phosphonoessigsäuretriäthylester der Formel 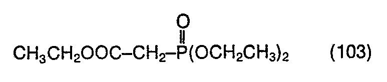 werden in 120 ml Dimethylformamid suspendiert und bei 30 °C unter Rühren und Stickstoff innerhalb 30 Minuten mit 18,0 g einer 30%igen methanolischen Natriummethylat-Lösung versetzt, wobei die Reaktionstemperatur bis 40 °C steigt. Es entsteht zuerst eine fast klare Lösung, aus welcher das Reaktionsprodukt gegen Ende der Zugabe der Natriummethylat-Lösung als dicker kristalliner Brei ausfällt. Das Reaktionsgemisch wird nun bei 30 °C unter Stickstoff vier Stunden weitergerührt, dann bei 0 °C mit 12 ml Eisessig neutralisiert und mit 200 ml Wasser verdünnt. Das Reaktionsprodukt wird abgenutscht, mit Wasser neutral gewaschen und unter Vakuum bei 80 °C bis zum konstanten Gewicht getrocknet. Man erhält 18 g (etwa 98,5% der Theorie) der Verbindung der Formel 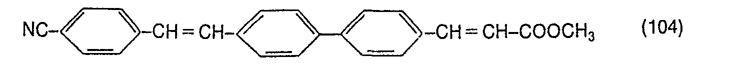 als hellgelbes kristallines Pulver mit einem Schmelzpunkt von 221 bis 226 °C.

Nach zweimaligem Umkristallisieren aus Chlorbenzol unter Zuhilfenahme von Aktivkohle erhält man 14 g der Verbindung der Formel (104) in Form von blassgelben Nadeln mit einem Schmelzpunkt von 230 bis 232 °C.

### Beispiel 2:

Wiederholt man Beispie) 1b), verwendet jedoch an Stelle einer methanolischen Natriummethylat-Lösung eine äthanolische Natriumäthylat-Lösung (erhalten durch Auflösen von 2,3 g Natrium in 50 ml wasserfreiem Äthanol), dann erhält man 18,2 g (etwa 95,9% der Theorie) der Verbindung der Formel 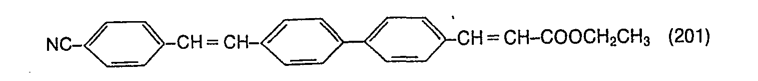 als gelbes kristallines Pulver mit einem Schmelzpunkt von 209 bis 215°C.

Nach dreimaligem Umkristallisieren aus Chlorbenzol unter Zuhilfenahme von Aktivkohle erhält man 10 g der Verbindung der Formel (201) in Form von blassgelben Nädelchen mit einem Schmelzpunkt von 219 bis 222 °C.

### Beispiel 3:

61,9 des 4-(4-Cyanostyryl)-biphenyl-4'-aldehyds der Formel (102) und 30 g wasserfreie Malonsäure werden in 200 ml trockenem Pyridin und 0,5 ml Piperidin während 20 Stunden unter Rühren am Rückfluss erhitzt. Das Reaktionsgemisch wird dann auf Raumtemperatur abgekühlt, unter Rühren mit 500 ml Wasser verdünnt und der Niederschlag abgenutscht. Das Nutschgut wird mit verdünnter Salzsäure, Wasser und Methanol gewaschen und unter Vakuum bei 80 °C bis zum konstanten Gewicht getrocknet. Man erhält 70,1 g (etwa 100% der Theorie) der Verbindung der Formel 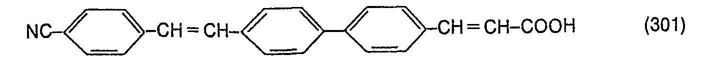 als hellgelbes kristallines Pulver mit einem Schmelzpunkt von über 300 °C.

Nach einmaligem Umkristallisieren aus Dimethylformamid erhält man 36,2 g der Verbindung der Formel (301) in Form von blassgelben Nädelchen mit einem Schmelzpunkt über 300 °C.

### Beispiel 4:

18,1 g der Säure der Formel (301) werden in 200 ml Chlorbenzol, 50 ml Thionylchlorid und 0,5 ml Dimethylformamid während zwei Stunden unter Rühren am Rückfluss erhitzt. Aus dem Reaktionsgemisch werden, nach Verdünnen mit 150 ml Chlorbenzol, 200 ml abdestilliert. Zur erhaltenen Lösung des Säurechlorids der Formel 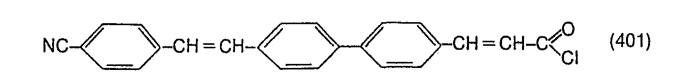 werden nun bei Rückflusstemperatur 50 ml n-Propanol zugetropft. Das Reaktionsgemisch wird während 16 Stunden am Rückfluss gehalten, dann mit 10 g Aktivkohle entfärbt und auf etwa 70 ml eingeengt. Das in der Kälte ausgeschiedene Reaktionsprodukt wird abgenutscht und unter Vakuum bei 80 °C getrocknet. Man erhält 11,4 g (etwa 57,9% der Theorie) der Verbindung der Formel 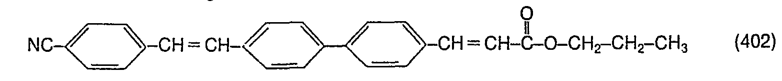 als gelbes kristallines Pulver mit einem Schmelzpunkt von 157 bis 160 °C.

Nach zweimaligem Umkristallisieren aus Toluol unter Zuhilfenahme von Aktivkohle erhält man 7,1 g der Verbindung der Formel (402) in Form von blassgelben Nadeln mit einem Schmelzpunkt von 159 bis 161 °C.

### Beispiel 5:

Wiederholt man Beispiel 4, verwendet jedoch an Stelle von n-Propanol Isopropanol, dann erhält man 15,3 g (etwa 77,8% der Theorie) der Verbindung der Formel 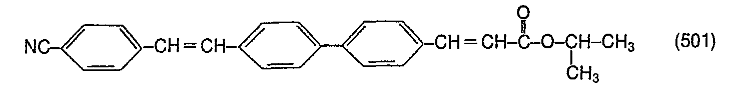 als hellgelbes kristallines Pulver mit einem Schmelzpunkt von 201 bis 206 °C.

Nach zweimaligem Umkristallisieren aus Toluol unter Zuhilfenahme von Aktivkohle erhält man 5,6 g der Verbindung der Formel (501) in Form von blassgelben Kristallen mit einem Schmelzpunkt von 209 bis 210 °C.

### Beispiel 6:

18,3 g des Methylesters der Formel (104) (siehe Beispiel 1b), 60 g Äthylenglykol-monomethyläther und 250 ml Dichlorbenzol werden unter Rühren auf 120 °C erhitzt und bei dieser Temperatur mit 1 ml Tetrabutyl-orthotitanat-monomer versetzt, wobei eine klare Lösung entsteht. Das Reaktionsgemisch wird während zwei Stunden bei 130 °C weitergerührt und dann auf etwa 70 ml eingeengt. Das in der Kälte ausgeschiedene Reaktionsproduktwird abgenutscht und unter Vakuum bei 80 °C getrocknet. Man erhält 15,5 g (etwa 75,7% der Theorie) der Verbindung der Formel 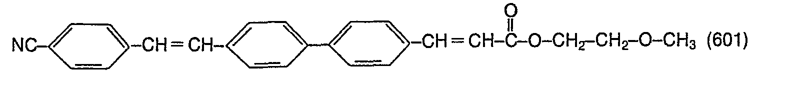 als hellgelbes kristallines Pulver mit einem Schmelzpunkt von 145 bis 149 °C.

Nach zweimaligem Umkristallisieren aus Chlorbenzol unter Zuhilfenahme von Aktivkohle erhält man 10 g der Verbindung der Formel (601) in Form von blassgelben Nädelchen mit einem Schmelzpunkt von 149 bis 151 °C.

In analoger Weise werden, ausgehend von der Verbindung der Formel (104), durch Umsetzung mit dem entsprechenden Alkohol die in der nachfolgenden Tabelle I angeführten Verbindungen der allgemeinen Formel 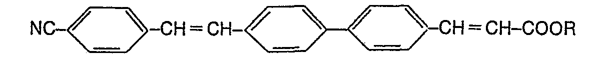 hergestellt.

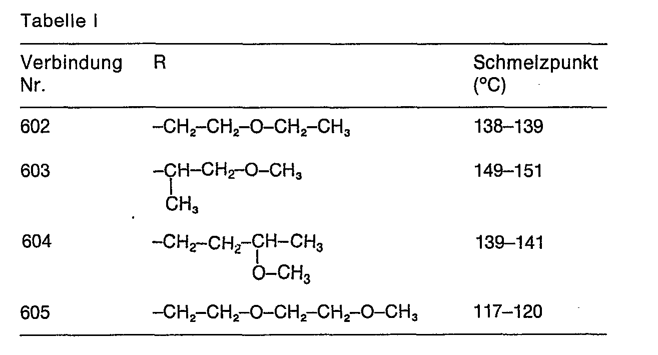

### Beispiel 7:

12,3 des 4-(4-Cyanostyryl)-biphenyl-4'-aldehyds der Formel (102) und 7 g Cyanmethylphosphonsäurediäthylester der Formel 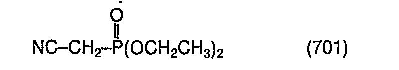 werden in 120 ml Dimethylformamid wie im Beispiel 1b) beschrieben umgesetzt.

Man erhält 12,5 g (etwa 94,6% der Theorie) der Verbindung der Formel 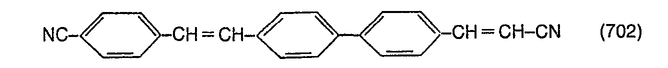 als gelbes kristallines Pulver mit einem Schmelzpunkt von 195 bis 202 °C.

Nach dreimaligem Umkristallisieren aus Chlorbenzol unter Zuhilfenahme von Aktivkohle erhält man 6,5 der Verbindung der Formel (702) in Form von blassgelben Kristallen mit einem Schmelzpunkt von 203 bis 206 °C.

### Beispiel 8:

a) 63 g Biphenyl-4,4'-dialdehyd werden in 500 ml wasserfreiem Methanol suspendiert und bei 20 bis 25 °C unter Rühren und Stickstoff innerhalb 15 Minuten mit 108 g einer 30%igen methanolischen Natriummethylat-Lösung versetzt. Es entsteht eine fast klare Lösung, die bei 20 bis 25 °C unter Rühren und Stickstoff innerhalb 30 Minuten mit 76 g des Phosphonates der Formel 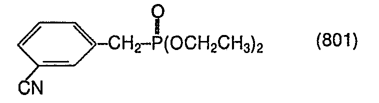 versetzt wird, wobei das Reaktionsprodukt langsam kristallin ausfällt. Die entstandene kristalline Suspension wird nun 20 Stunden bei 20 bis 25 °C unter Stickstoff weitergerührt, der Niederschlag abgenutscht, mit etwa 70 ml wasserfreiem Methanol gewaschen und unter Vakuum bei 80 °C bis zum konstanten Gewicht getrocknet. Man erhält 65,5 g (etwa 70,6% der Theorie) des Aldehyds der Formel 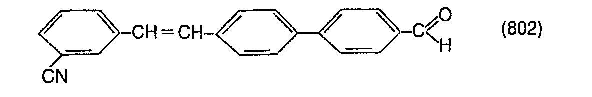 in Form eines gelben kristallinen Pulvers mit einem Schmelzpunkt von 137 bis 144 °C. Nach zweimaligem Umkristallisieren aus Chlorbenzol unter Zuhilfenahme von Aktivkohle erhält man 31,5 g des Aldehyds der Formel (802) in Form von gelben Nädelchen mit einem Schmelzpunkt von 165 bis 167 °C.

Das als Ausgangsmaterial verwendete Phosphonat der Formel (801) wird gemäss Beispiel 2 der britischen Patentschrift 920 988 hergestellt.

b) 15,5 g des 4-(3-Cyanostyryl)-biphenyl-4'-aldehyds der Formel (802) und 8,9 g Cyanmethylphosphonsäurediäthylester der Formel (701) werden in 100 ml Dimethylformamid wie in Beispiel 1b) beschrieben umgesetzt. Man erhält auf diese Weise 15,6 g (etwa 93,8% der Theorie) der Verbindung der Formel 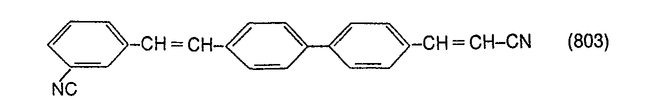 als blassgelbes kristallines Pulver mit einem Schmelzpunkt von 204 bis 210 °C.

Nach dreimaligem Umkristallisieren aus Chlorbenzol unter Zuhilfenahme von Aktivkohle erhält man 11 g der Verbindung der Formel (803) in Form von blassgelben Kristallen mit einem Schmelzpunkt von 210 bis 212 °C.

### Beispiel 9:

a) 84 g Biphenyl-4,4'-dialdehyd werden in 920 ml wasserfreiem Äthanol suspendiert, auf Rückflusstemperatur erhitzt und dann auf Raumtemperatur abgekühlt. Die erhaltene feine Suspension wird bei Raumtemperatur unter Rühren und Stickstoff innerhalb 15 Minuten mit 144 g einer 30%igen methanolischen Natriummethylat-Lösung versetzt. Es entsteht eine fast klare Lösung, die bei Raumtemperatur unter Rühren und Stickstoff innerhalb 15 Minuten mit einer Lösung von 101,2 g des Phosphonates der Formel 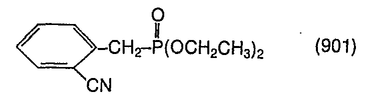 in 80 ml wasserfreiem Äthanol versetzt wird, wobei das Reaktionsprodukt langsam kristallin ausfällt. Die entstandene kristalline Suspension wird nun 20 Stunden bei Raumtemperatur unter Stickstoff weitergerührt; dann werden die Kristalle abgenutscht, mit etwa 100 ml wasserfreiem Äthanol gewaschen und unter Vakuum bei 80 °C bis zum konstanten Gewicht getrocknet.

Man erhält 80,5 g (etwa 65,1 % der Theorie) des Aldehyds der Formel 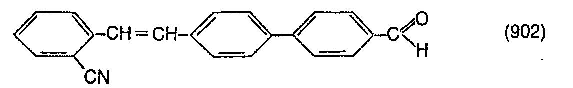 in Form eines blassgelben kristallinen Pulvers mit einem Schmelzpunkt von 161 bis 168 °C.

Nach zweimaligem Umkristallisieren aus Chlorbenzol unter Zuhilfenahme von Aktivkohle erhält man 61 g der Verbindung der Formel (902) in Form von blassgelben Nädelchen mit einem Schmelzpunkt von 169 bis 171 °C.

Das als Ausgangsmaterial verwendete Phosphonat der Formel (901) wird in Analogie zu Beispiel 1 der deutschen Offenlegungsschrift 1 921 466 hergestellt und durch Destillation gereinigt (Kpₒ,₃ₛ: 136-138 °C).

b) 15,5 g des 4-(2-Cyanostyryl)-biphenyl-4'-aldehyds der Formel (902) und 11,2 g Phosphonoessigsäuretriäthylester der Formel (103) werden in 100 ml Dimethylformamid wie in Beispiel 1b) beschrieben umgesetzt.

Man erhält auf diese Weise 17,5 g (etwa 95,7% der Theorie) der Verbindung der Formel 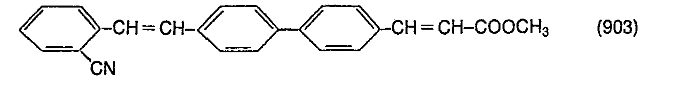 als hellgelbes kristallines Pulver mit einem Schmelzpunkt von 154 bis 161 °C.

Nach zweimaligem Umkristallisieren aus Chlorbenzol unter Zuhilfenahme von Aktivkohle erhält man 10 g der Verbindung der Formel (903) in Form von blassbelben Nadeln mit einem Schmelzpunkt von 167 bis 169 °C.

### Beispiel 10:

Wiederholt man Beispiel 9b), verwendet jedoch an Stelle einer methanolischen Natriummethylat-Lösung eine äthanolische Natriumäthylat-Lösung (erhalten durch Auflösen von 2,3 g Natrium in 50 ml wasserfreiem Äthanol), dann erhält man 17,9 g (etwa 94,3% der Theorie) der Verbindung der Formel 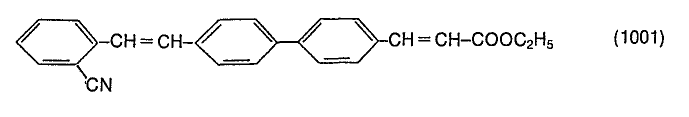 als gelbes kristallines Pulver mit einem Schmelzpunkt von 144 bis 152 °C.

Nach zweimaligem Umkristallisieren aus Chlorbenzol unter Zuhilfenahme von Aktivkohle erhält man 7,2 der Verbindung der Formel (1001) in Form von blassgelben Kristallen mit einem Schmelzpunkt von 175 bis 180 °C.

### Beispiel 11:

15,5 des 4-(2-Cyanostyryl)-biphenyl-4'-aldehyds der Formel (902) und 8,9 g Cyanmethylphosphonsäurediäthylester der Formel (701) werden in 100 ml Dimethylformamid wie in Beispiel 1b) beschrieben umgesetzt.

Man erhält auf diese Weise 16,1 g (etwa 96,8% der Theorie) der Verbindung der Formel 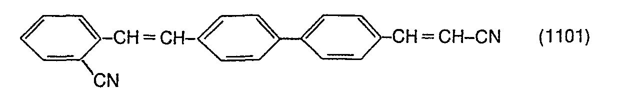 als gelbes kristallines Pulver mit einem Schmelzpunkt von 185 bis 202 °C.

Nach dreimaligem Umkristallisieren aus Chlorbenzol unter Zuhilfenahme von Aktivkohle erhält man 7,5 der Verbindung der Formel (1101) in Form von blassgelben Kristallen mit einem Schmelzpunkt von 207 bis 211 °C.

### Beispiel 12:

a) 42g g Biphenyl-4,4'-dialdehyd werden in 400 ml wasserfreiem Methanol suspendiert und bei 20 bis 25 °C unter Rühren und Stickstoff innerhalb 15 Minuten mit 72 g einer 30%igen methanolischen Natriummethylat-Lösung versetzt. Es entsteht eine fast klare Lösung, die bei 20 bis 25 °C unter Rühren und Stickstoff innerhalb 15 Minuten mit einer Lösung von 52 g des Phosphonates der Formel 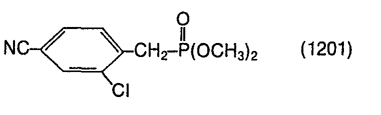 in 100 ml wasserfreiem Methanol versetzt wird, wobei das Reaktionsprodukt sofort kristallin ausfällt. Der entstandene dicke kristalline Reaktionsbrei wird nun 6 Stunden bei 20 bis 25 °C unter Stickstoff weitergerührt, dann abgenutscht, mit etwa 100 ml wasserfreiem Methanol gewaschen und unter Vakuum bei 80 °C bis zum konstanten Gewicht getrocknet. Man erhält 62,5 g (etwa 90,9% der Theorie) des Aldehyds der Formel 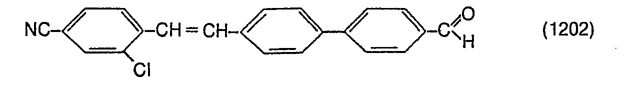 in Form eines gelben kristallinen Pulvers mit einem Schmelzpunkt von 139 bis 164 °C. Nach zweimaligem Umkristallisieren aus Chlorbenzol unter Zuhilfenahme von Aktivkohle erhält man 30,2 g der Verbindung der Formel (1202) in Form von gelben Nadeln mit einem Schmelzpunkt von 152 bis 154 °C.

b) 17 g des 4-(2-Chlor-4-cyanostyryl)-biphenyl-4'-aldehyds der Formel (1202) und 9 g Cyanmethylphosphonsäurediäthylester der Formel (701) werden in 100 ml Dimethylformamid wie in Beispiel 1b) beschrieben umgesetzt.

Man erhält 17,0 g (etwa 92,9% der Theorie) der Verbindung der Formel 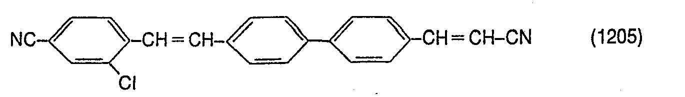 als gelbes kristallines Pulver mit einem Schmelzpunkt von 150 bis 158 °C.

Nach zweimaligem Umkristallisieren aus Dimethylformamid erhält man 5 g der Verbindung der Formel (1205) in Form von blassgelben Kristallen mit einem Schmelzpunkt von 186 bis 189 °C.

Das als Ausgangsmaterial verwendete Phosphonat der Formel (1201) wird wie folgt hergestellt:
360g g 3-Chlor-4-methylanilin werden gemäss A. Goldberg und W. Kelly, J. Chem. Soc. 1947, 637-641 diazotiert. Die erhaltene wässrige Diazoniumsalzlösung wird in eine aus Natriumcyanid und Nickel(II)-chlorid in Wasser hergestellte Lösung bei Siedetemperatur eingetragen. Man erhält 216,4 g (etwa 57,1 % der Theorie) des Nitrils der Formel 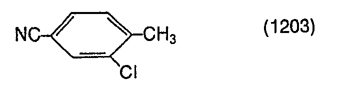 in Form eines weissen kristallinen Pulvers mit einem Schmelzpunkt von 47 bis 49 °C.
151,6 g des Nitrils der Formel (1203) werden in 700 ml Tetrachlorkohlenstoff aufgenommen und mit 187 g N-Bromsuccinimid und 2 g Dibenzoylperoxid versetzt. Die erhaltene Suspension wird 24 Stunden lang auf Rückflusstemperatur erhitzt, dann auf Raumtemperatur abgekühlt und abgenutscht. Das Nutschgut wird mit 150 ml Tetrachlorkohlenstoff gewaschen und das Flltrat unter Vakuum zur Trockne eingeengt. Man erhält 230,8 g eines gelben Öls, das nach einmaligem Umkristallisieren aus Äthanol 125,8 g (etwa 54,6% der Theorie) der Verbindung der Formel 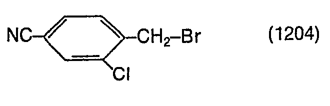 in Form eines weissen kristallinen Pulvers mit einem Schmelzpunkt von 83 bis 85 °C liefert.
208 g geschmolzenes Bromid der Formel (1204) werden innerhalb einer Stunde unter Rühren bei 100 bis 105 °C zu 350 ml Trimethylphosphit zugetropft, wobei aus dem Reaktionsgemisch Methylchlorid entweicht. Das Reaktionsgemisch wird nun zuerst eine Stunde bei 110 bis 115 °C, dann zwei Stunden bei 120 bis 125 °C weitergerührt, wobei ein Teil des überschüssigen Trimethylphosphits abdestilliert. Das erhaltene rohe Reaktionsgemisch wird unter Vakuum vom noch vorhandenen überschüssigen Trimethylphosphit befreit. Man erhält 220 g (etwa 93,9% der Theorie) der Verbindung der Formel (1201) in Form einer leicht braun gefärbten kristallinen Masse mit einem Schmelzpunkt von 85 bis 87 °C. Dieses rohe Phosphonat wird durch Destillation gereinigt. Man erhält 197 g reines Phosphonat der Formel (1201) in Form von weissen Kristallen mit einem Schmelzpunkt von 89 bis 90 °C (KP_{0.04}: 150-152 °C).

### Beispiel 13:

a) 42 g Biphenyl-4,4'-dialdehyd werden mit 52 g des Phosphonates der Formel 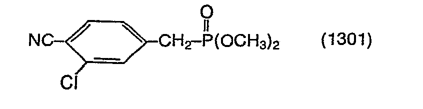 wie im Beispiel 12a) beschrieben umgesetzt.

Man erhält 55,2 g (etwa 80,3% der Theorie) des Aldehyds der Formel 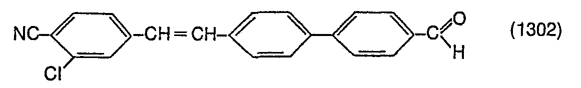 in Form eines gelben kristallinen Pulvers mit einem Schmelzpunkt von 184 bis 205 °C.

Nach zweimaligem Umkristallisieren aus Chlorbenzol unter Zuhilfenahme von Aktivkohle erhält man 35,5 g der Verbindung der Formel (1302) in Form von gelben Nadeln mit einem Schmelzpunkt von 198 bis 200 °C.

b) 17 g des 4-(3-Chlor-4-cyanostyryl)-biphenyl-4'-aldehyds der Formel (1302) und 9 g Cyanmethylphosphonsäuredimethylester der Formel (701) werden in 100 ml Dimethylformamid wie im Beispiel 1b) beschrieben umgesetzt.

Man erhält 17,2 g (etwa 93,8% der Theorie) der Verbindung der Formel 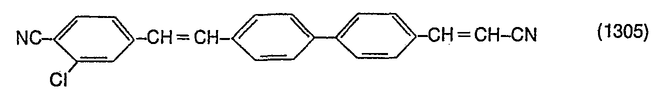 als gelbes kristallines Pulver mit einem Schmelzpunkt von 219 bis 225 °C.

Nach zweimaligem Umkristallisieren aus Chlorbenzol unter Zuhilfenahme von Aktivkohle erhält man 11 g der Verbindung der Formel (1305) in Form von blassgelben Kristallen mit einem Schmelzpunkt von 226 bis 227 °C.

Das als Ausgangsmaterial verwendete Phosphonat der Formel (1301) wird wie folgt hergestellt:
204 g 2-Chlor-4-methyl-anilin werden gemäss A. Goldberg und W. Kelly, J. Chem. Soc. 1947, 637-641, diazotiert und die bereitete wässrige Diazoniumsalz-Lösung wird mit Natriumcyanid wie im Beispiel 12 beschrieben umgesetzt.

Man erhält 88,9 g (etwa 40,7% der Theorie) des Nitrils der Formel 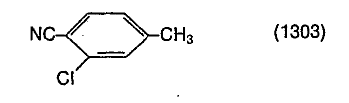 in Form eines weissen kristallinen Pulvers mit einem Schmelzpunkt von 60 bis 61 °C.

88 g des Nitrils der Formel (1303) werden mit 109 g N-Bromsuccinimid wie im Beispiel 12 beschrieben umgesetzt.

Man erhält 140 g eines gelben Öls, das nach einmaligem Umkristallisieren aus Äthanol 93 g (etwa 69,6% der Theorie) der Verbindung der Formel 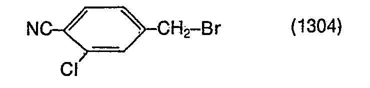 in Form eines weissen kristallinen Pulvers mit einem Schmelzpunkt von 41 bis 44 °C liefert.

93 g des Bromids der Formel (1304) werden mit 120 ml Trimethylphosphit wie im Beispiel 12 beschrieben umgesetzt.

Man erhält 102 g (etwa 97,4% der Theorie) der Verbindung der Formel (1301) in Form eines leicht braungefärbten Öls. Dieses rohe Phosphonat wird durch Destillation gereinigt. Man erhält 70,5 g reines Phosphonat der Formel (1301) in Form von weissen Kristallen mit einem Schmelzpunkt von 71 bis 73 °C (Kpₒ,₀₅:148-150 °C).

### Beispiel 14:

a) 42 g Biphenyl-4,4'-dialdehyd werden in 200 ml wasserfreiem Äthanol suspendiert und bei 20 bis 25 °C unter Rühren und Stickstoff innerhalb 15 Minuten mit 160 ml mit einer 2,5 molaren Natriumäthylat-Lösung versetzt. Es entsteht eine fast klare Lösung, die bei 20 bis 25 °C unter Rühren und Stickstoff innerhalb 15 Minuten mit 60 g des Phosphonates der Formel 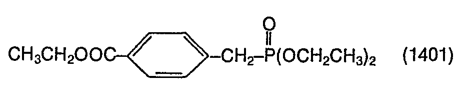 versetzt wird, wobei das Reaktionsprodukt sofort kristallin ausfällt. Der entstandene dicke kristalline Reaktionsbrei wird nun 6 Stunden bei 20 bis 25 °C unter Stickstoff weitergerührt, mit 200 ml Äthanol verdünnt, dann abgenutscht, mit etwa 100 ml Äthanol gewaschen und unter Vakuum bei 80 °C bis zur Gewichtskonstanz getrocknet.

Man erhält 62 g (etwa 87% der Theorie) des Aldehyds der Formel 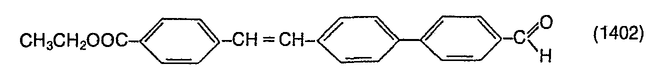 in Form eines gelben kristallinen Pulvers mit einem Schmelzpunkt von 176 bis 180 °C.

Nach zweimaligem Umkristallisieren aus Chlorbenzol unter Zuhilfenahme von Aktivkohle erhält man 44,5 g der Verbindung der Formel (1402) in Form von gelben Nadeln mit einem Schmelzpunkt von 180 bis 183 °C.

Das als Ausgangsmaterial verwendete Phosphonat der Formel (1401) wird analog Beispiel 2 der GB-PS 929 436 hergestellt und durch Destillation gereinigt (Kp_{0,25}: 181-185 °C).

b) 17,8 g des 4-(4-Carbäthoxystyryl)-biphenyl-4'-aldehyds der Formel (1402) und 9 g Cyanmethylphosphonsäurediäthylester der Formel (701) werden in 120 ml Dimethylformamid wie im Beispiel 1b) beschrieben umgesetzt, wobei an Stelle von 18,0 g einer 30%igen methanolischen Natriummethylat-Lösung 22 ml einer2,5 molaren Natriumäthylat-Lösung verwendet werden.

Man erhält 17,5 g (etwa 93% der Theorie) der Verbindung der Formel 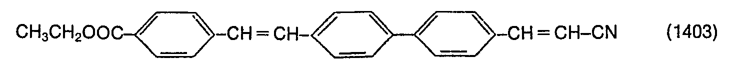 als gelbes kristallines Pulver mit einem Schmelzpunkt von 194 bis 200 °C.

Nach zweimaligem Umkristallisieren aus Chlorbenzol unter Zuhilfenahme von Aktivkohle erhält man 11,5 g der Verbindung der Formel (1403) in Form von blassgelben Kristallen mit einem Schmelzpunkt von 199 bis 201 °C.

In analoger Weise wird die Verbindung der Formel 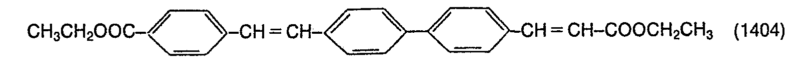 hergestellt.

### Beispiel 15:

17,8 g des 4-(4-Carbäthoxystyryl)-biphenyl-4'- aldehyds der Formel (1402) und 9 g Cyanmethylphosphonsäurediäthylester der Formel (701) werden in 120 ml Dimethylformamid in Gegenwart von 10 g einer 30%igen methanolischen Natriummethylat-Lösung wie im Beispiel 1b) beschrieben umgesetzt.

Man erhält 17,5 g (etwa 96% der Theorie) der Verbindung der Formel 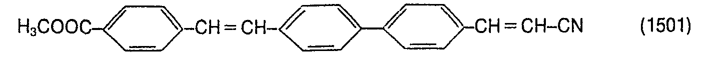 als gelbes kristallines Pulver mit einem Schmelzpunkt von 216 bis 225 °C.

Nach zweimaligem Umkristallisieren aus Chlorbenzol unter Zuhilfenahme von Aktivkohle erhält man 13 g der Verbindung der Formel (1501) in Form von blassgelben Kristallen mit einem Schmelzpunkt von 226 bis 228 °C.

In analoger Weise wird die Verbindung der Formel 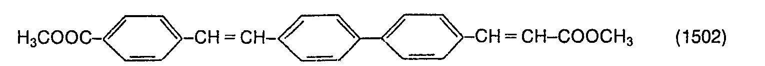 hergestellt.

### Beispiel 16:

15,2 g der Verbindung der Formel (1403) werden in 250 ml 1,2-Dichlorbenzol gelöst und die erhaltene klare Lösung wird mit 50 ml 2-Methoxy-äthanol und 1 ml Tetrabutyl-orthotitanat versetzt. Das Reaktionsgemisch wird zwei Stunden bei 130 °C gerührt, anschliessend auf 150 ml aufkonzentriert und bei Raumtemperatur unter Rühren auskristallisieren gelassen. Das Reaktionsprodukt wird abgenutscht, mit 50 ml Chlorbenzol gewaschen und unter Vakuum bei 100 °C bis zur Gewichtskonstanz getrocknet. Man erhält 15 g (etwa 94% der Theorie) der Verbindung der Formel 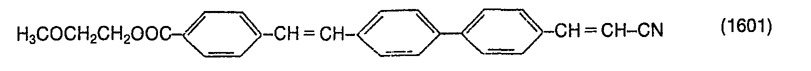 als gelbes kristallines Pulver mit einem Schmelzpunkt von 164 bis 170 °C.

Nach zweimaligem Umkristallisieren aus Chlorbenzol unter Zuhilfenahme von Aktivkohle erhält man 10,5 g der Verbindung der Formel (1601) in Form von blassgelben Kristallen mit einem Schmelzpunkt von 168 bis 170 °C.

In analoger Weise werden aus der Verbindung der Formel (1403) durch Umsetzung mit einem entsprechenden Alkohol (Umesterung) die Verbindungen der Formeln 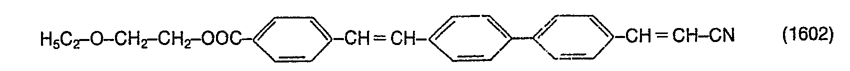 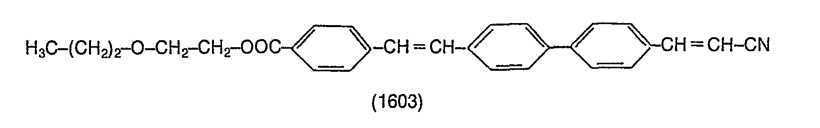 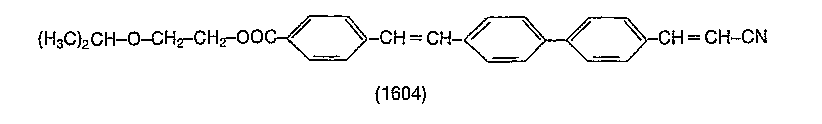 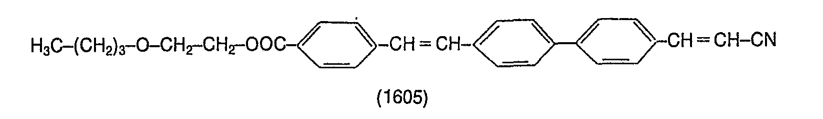 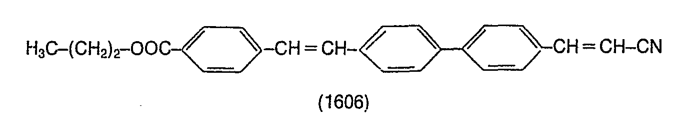 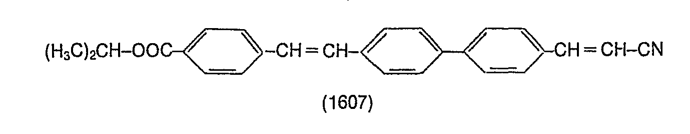 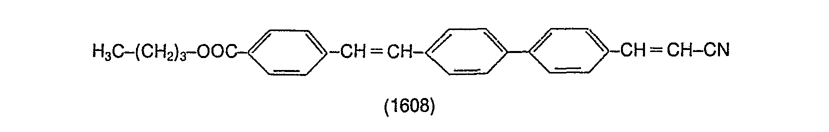 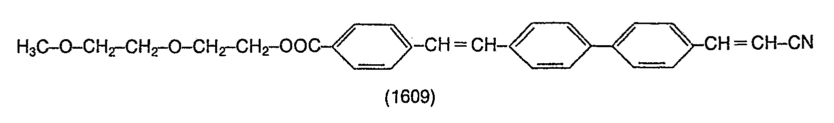 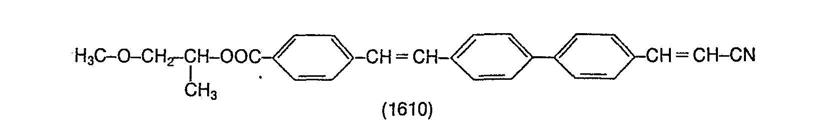 hergestellt.

### Beispiel 17:

1 g der Verbindung der Formel (702) wird in 1000 ml Wasser dispergiert. 7,5 ml dieser Dispersion werden 300 ml Wasser zugegeben, die 0,1 g eines Fettalkoholpolyglykoläthers enthalten. Zu dieser auf 60 °C erwärmten Aufhellerdispersion gibt man ein 15 g schweres Polyestergewebe. Man steigert die Temperatur innerhalb 15 bis 20 Minuten auf 120 °C und belässt bei dieser Temperatur 30 Minuten lang. Dann kühlt man innerhalb von 10 bis 15 Minuten auf 60 °C ab. Sodann wird das Gewebe 2 Minuten lang in fliessendem kaltem Wasser gespült und anschliessend 20 Minuten lang bei 60 °C getrocknet.

Das so behandelte Gewebe weist einen ausgezeichneten Aufhelleffekt von guter Lichtechtheit auf.

Verwendet man in dieser Vorschrift an Stelle der genannten Verbindung der Formel (702) eine solche der Formel (104), (201) oder (601), so erhält man ähnlich gute Aufhelleffekte.

### Beispiel 18:

Polyestergewebe wird bei Raumtemperatur mit einer wässrigen Dispersion foulardiert, die im Liter 0,5 g der Verbindung der Formel (702) sowie 1 g eines Anlagerungsproduktes aus etwa 8 Mol Äthylenoxid an 1 Mol p-tert.-Octylphenol enthält. Die Flüssigkeitsaufnahme beträgt 60 bis 70%. Das Gewebe wird bei 100 °C getrocknet und anschliessend 30 Sekunden lang auf 180 °C erhitzt.

Das so behandelte Gewebe weist einen ausgezeichneten Aufhelleffekt von guter Lichtechtheit auf.

Verwendet man in dieser Vorschrift an Stelle der genannten Verbindung der Formel (702) eine solche der Formel (104), (201) oder (601), so erhält man ähnlich gute Aufhelleffekte.

### Beispiel 19:

Wiederholt man Beispiel 17 und 18, setzt jedoch an Stelle der Aufhellerverbindung der Formel (702) eine äquivalente Menge einer der Verbindungen der Formeln (301), (402), (501), (602) bis (605), (803), (903), (1001), (1101), (1205), (1305), (1403), (1404), (1501), (1502) oder (1601) bis (1610) ein, so erhält man ebenfalls gut aufgehelltes Polyestergewebe.

### Beispiel 20:

Polyestergewebe wird bei Raumtemperatur mit einer wässrigen Dispersion foulardiert, die im Liter 0,5 g einer Aufhellermischung, bestehend aus 2 Teilen der Verbindung der Formel (702) und 1 Teil der Verbindung der Formel 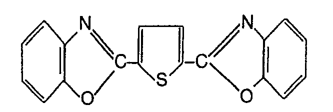 sowie 1 g eines Anlagerungsproduktes aus etwa 8 Mol Äthylenoxid an 1 Mol p-tert.-Octylphenol enthält. Die Flüssigkeitsaufnahme beträgt 60-70%. Das Gewebe wird bei 100 °C getrocknet und anschliessend 30 Sekunden lang auf 200 °C erhitzt.

Das so behandelte Gewebe weist einen ausgezeichneten Aufhelleffekt von guter Lichtechtheit auf.

### Beispiel 21:

Man behandelt Polyestergewebe nach der in Beispiel 20 angegebenen Vorschrift, ersetzt jedoch die dort verwendete Aufhellermischung durch eine der in der nachfolgenden Tabelle II angeführten Aufhellermischungen A-I.

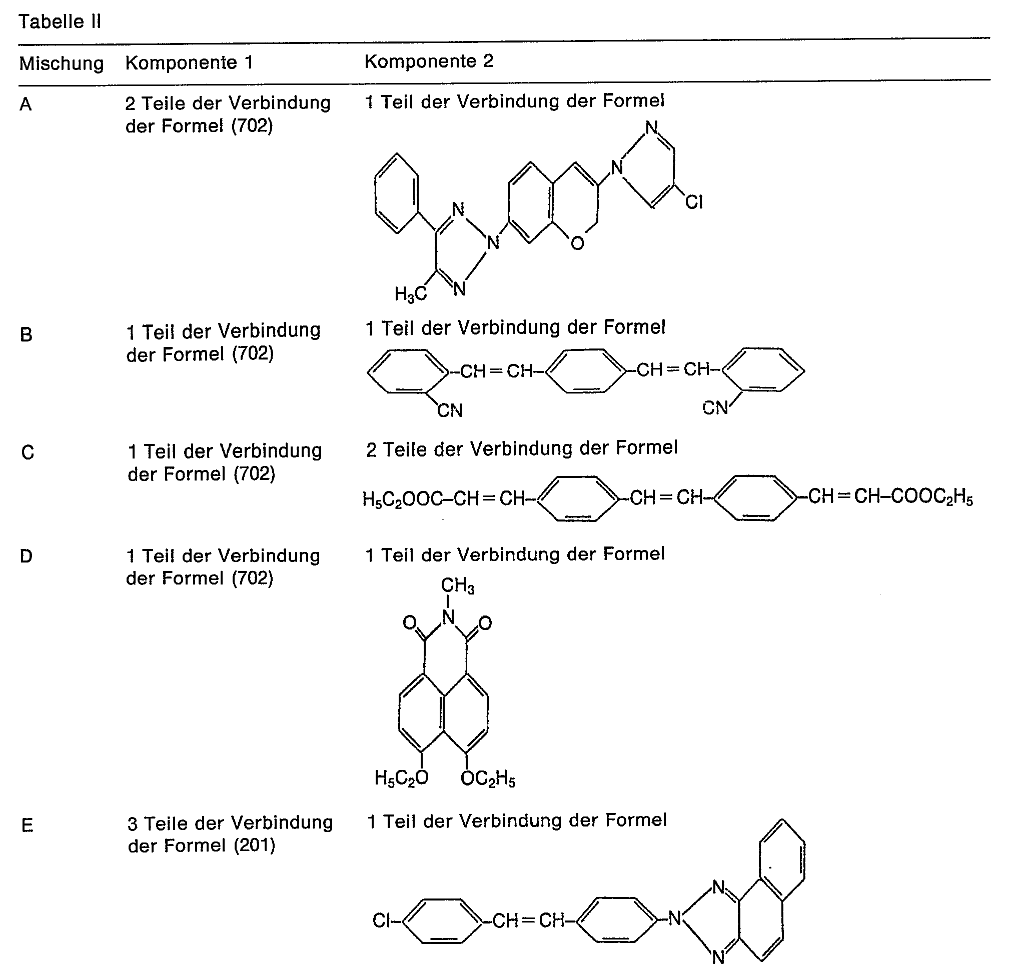

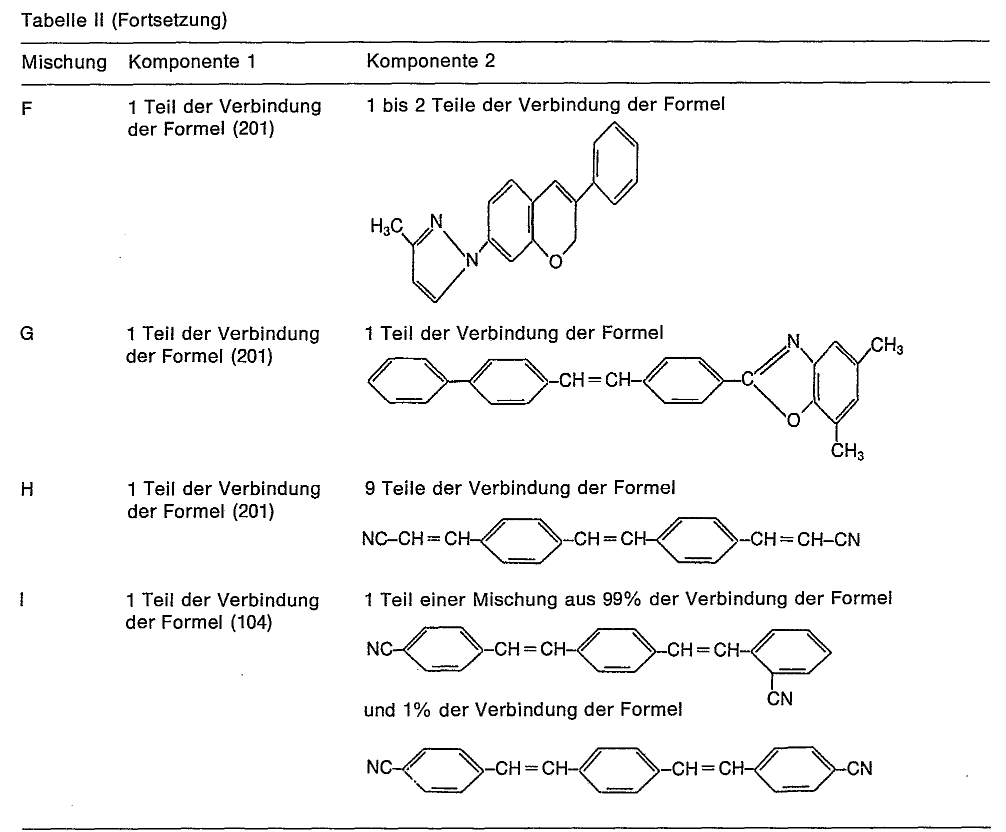

Das mit der jeweiligen Aufheller-Mischung behandelte Polyestergewebe weist jeweils einen ausgezeichneten Aufhelleffekt auf.

### Beispiel 22:

Ein Polyamid 6,6-Webtricot wird auf einem Färbeapparat bei einem Flottenverhältnis von 1 : 20 mit einem wässrigen Bad behandelt, welches 0,2%, bezogen auf das Gewicht des Gewebes, einer Verbindung der Formel (702), (601) oder (201), 3 g/I eines Gemisches von 60 Gewichtsteilen Natriumhydrosulfit und 40 Gewichtsteilen Natriumpyrophosphat und 1 ml/I 80%ige Essigsäure enthält. Man erwärmt das Bad innerhalb von 30 Minuten auf 97 °C, behält es während 30 Minuten bei dieser Temperatur und kühlt dann innerhalb von 15 Minuten auf 40 °C ab. Das Gewebe wird anschliessend in fliessendem deionisiertem Wasser gespült und bei 180 °C mit dem Büqeleisen getrocknet.

Das so behandelte Polyamidgewebe weist in allen 3 Fällen einen hohen Aufhelleffekt auf.

Verwendet man in obiger Vorschrift an Stelle der Verbindungen der Formeln (702), (601) und (201) äquivalente Mengen der Verbindungen der Formeln (104), (301), (402), (501), (602) bis (605), (803), (903), (1001), (1101), (1205), (1305), (1403), (1404), (1501), (1502) oder (1601) bis (1610), so kommt man ebenfalls zu gut aufgehelltem Polyamidgewebe.

Behandelt man nach obiger Vorschrift Polyamidgewebe an Stelle der Verbindungen der Formeln (702), (601) oder (201) mit der gleichen Menge einer Mischung aus 1 Teil der Verbindung der Formel (1001) und 1 Teil der Verbindung der Formel 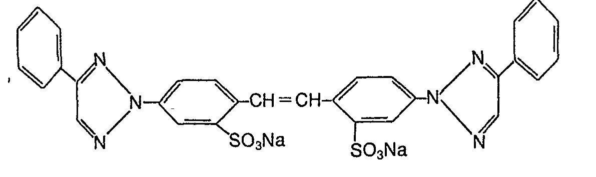 oder einer Mischung aus 1 Teil der Verbindung der Formel (1001) und 1 Teil der Verbindung der Formel 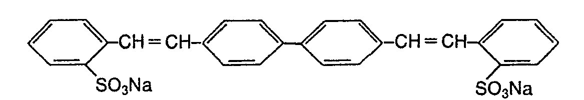 so erhält man ebenfalls Polyamidgewebe mit einem ausgezeichneten Weissgrad.

### Beispiel 23:

Vom Aufheller der Formel (201), (601) oder (702) wird 1 g jeweils in 1000 ml Wasser dispergiert. 1,5 ml dieser Dispersion werden 100 ml Wasser zugegeben, die 0,12 ml Ameisensäure 85% und 0,06 g Alkylpolyglycoläther enthalten. Zu dieser auf 60 °C erwärmten Aufhellerdispersion gibt man ein 3 g schweres Polyacrylnitrilgewebe. Man steigert die Temperatur innerhalb von 10 bis 15 Minuten auf 95 bis 97 °C und belässt bei dieser Temperatur 1 Stunde lang. Sodann wird das Gewebe 2 Minuten lang in fliessendem kaltem Wasser gespült und anschliessend 20 Minuten lang bei 60 °C getrocknet.

Das so behandelte Gewebe weist einen guten Aufhelleffekt auf.

### Beispiel 24:

Ein Triacetatgewebe wird auf einem Färbeapparat bei einem Flottenverhältnis von 1 : 20 mit einem wässrigen Bad behandelt, welches 0,1%, bezogen auf das Warengewicht, der Verbindung der Formel (104), (201), (702), (1001) oder (1205) und 1 g/Liter des Kondensationsproduktes von 35 Mol Äthylenoxid mit 1 Mol Stearylalkohol enthält. Man erwärmt nun das Bad innerhalb 30 Minuten von 40 auf 97 °C, behält es während 30 Minuten auf dieser Temperatur und kühlt es dann innerhalb 15 Minuten auf 30 °C ab. Das Gewebe wird anschliessend in fliessendem, deionisiertem Wasser gespült und bei 60 °C getrocknet. Das so behandelte Triacetatgewebe weist einen hohen Aufhelleffekt auf.

### Beispiel 25:

Ein Acetatsatingewebe wird auf einem Färbeapparat bei einem Flottenverhältnis von 1 : 20 mit einem wässrigen Bad behandelt, welches 0,1%, bezogen auf das Warengewicht, der Verbindung der Formel (104), (201), (601) oder (702), 1 g/Liter des Kondensationsproduktes von 35 Mol Äthylenoxid mit 1 Mol Stearylalkohol und 0,5 ml/Liter Essigsäure 80% enthält. Man erwärmt nun das Bad innerhalb 30 Minuten von 40 auf 80 °C, behält es während 30 Minuten bei dieser Temperatur und kühlt es dann innerhalb 15 Minuten auf 20 °C ab. Das Gewebe wird anschliessend in fliessendem, deionisiertem Wasser gespült und bei 60 °C getrocknet. Das so behandelte Acetatsatin-Gewebe zeichnet sich durch einen hohen Aufhelleffekt aus.

### Beispiel 26:

1000 g Polyestergranulat vom Typ Äthylenglykolterephthalat, enthaltend 0,5% Ti0₂ (Anatastyp), werden mit 0,5 g einer Verbindung der Formel (201), (601), (1501) oder (702) in einem Rhönradmischer gemischt und das so behandelte Granulat in einer Extruderspinnanlage bei 280 °C zu einem Multifilament versponnen. Die entstandenen Fäden zeigen einen ausgezeichneten Aufhelleffekt von guter Lichtechtheit.

### Beispiel 27:

100 Teile Polystyrol, enthaltend ca. 1,5% Ti0₂ (Rutiltyp), werden mit 0,05 Teilen einer Verbindung der Formel (104), (301), (402) oder (702) trokken vermischt und auf einem Extruder bei 180 °C zu einem aufgehellten Granulat verarbeitet. Das Granulat wird mit Hilfe einer Kolbenspritzgussmaschine zu Plättchen verformt. Die so erhaltenen Plättchen weisen einen starken Aufhelleffekt von guter Lichtechtheit auf.

### Beispiel 28:

Eine innige Mischung aus 65 Teilen Polyvinylchlorid (Suspensionstyp), 32 Teilen Dioctylphthalat, 3 Teilen eines epoxidierten Sojabohnenöls, 1,5 Teilen eines Stabilisators, 0,5 Teilen eines Costabilisators, 5 Teilen Ti0₂ (Rutiltyp) und 0,05 Teilen einer Verbindung der Formel (501), (1001), (1404) oder (1601) werden auf einem Kalander bei 150 °C zu einer Folie ausgewalzt. Die erhaltene Folie weist einen starken Aufhelleffekt von guter Lichtechtheit auf.

## Patentansprüche

1. 4-Styryl-4'-vinylbiphenyle der Formel 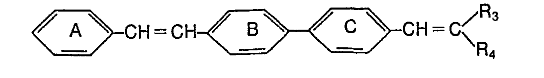 worin die Benzolringe A, B und C durch nicht-chromophore Substituenten substituiert sein können, R₃ einen nicht-chromophoren Substituenten 2. Ordnung und R₄ Wasserstoff oder einen nicht-chromophoren Substituenten, der jedoch kein Substituent 2. Ordnung sein kann, bedeuten.

2. 4-Styryl-4'-vinylbiphenyle nach Anspruch 1 der Formel 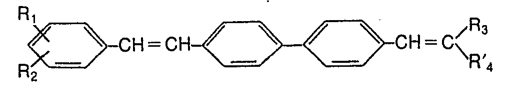 worin R₁ und R₂ jeweils Wasserstoff oder einen nicht-chromophoren Substituenten oder beide zusammen in ortho-Stellung die Ergänzung zu einem anellierten Ring, R₃ einen nicht-chromophoren Substituenten 2. Ordnung und R₄ Wasserstoff oder gegebenenfalls nicht-chromophor substituiertes Alkyl oder Alkenyl bedeuten.

3. 4-Styryl-4'-vinylbiphenyle nach Anspruch 1 der Formel 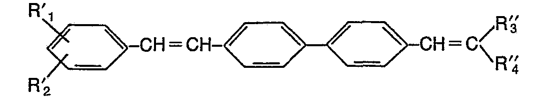 worin R₁' Wasserstoff, Halogen, unsubstituiertes oder nicht-chromophor substituiertes Alkyl, Alkoxy oder Alkylsulfonyl, unsubstituiertes oder nicht-chromophorsubstituiertes Phenyl, Phenylalkyl oder Phenylsulfonyl, Phenoxy, Phenylalkoxy, Cyano, eine Gruppe der Formel -COOY₁, -CONY₁Y₂ oder -SO₂NY₁Y₂, worin Y₁ und Y₂ unabhängig voneinander für Wasserstoff, Alkenyl, Propargyl, Cycloalkyl mit 5 oder 6 Ring-C-Atomen, unsubstituiertes oder nicht-chromophor substituiertes Alkyl, Phenyl, Phenylalkyl, oder Y₁ und Y₂ zusammen mit dem Stickstoffatom, an das sie gebunden sind, für einen 5-oder 6-gliedrigen gesättigten heterocyclischen Ring stehen, der zusätzlich 1 oder 2 Heteroatome als Ringglieder enthalten und durch Alkylgruppen substituiert sein kann, oder worin Y₁ in der Gruppe -COOY₁ ausserdem für ein salzbildendes Kation stehen kann; oder
eine Gruppe der Formel 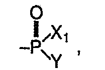 worin X₁ und Y unabhängig voneinander für Halogen, Alkyl, Alkenyl, Phenyl, Phenylalkyl, Hydroxy, Alkoxy, Phenylalkoxy, Cycloalkoxy, Phenoxy, Amino, Mono- oder Dialkylamino, Phenylalkylamino, Acylamino, Phenylamino, Cycloalkylamino, Morpholino, Piperidino oder Pyrrolidino stehen; oder zusammen mit R2 in ortho-Stellung den Rest der Formel -CH=CH-CH=CH-, -O-CH₂-O- oder -O-CH₂CH₂-O-, R₂' Wasserstoff, Halogen, unsubstituiertes oder nicht-chromophor substituiertes Alkyl oder Alkoxy oder zusammen mit Rᵢ' in ortho-Stellung den Rest der Formel -CH = CH-CH = CH-, -O-CH2-O- oder -O-CH₂-CH₂-O-, R3" Alkylsulfonyl, Phenylsulfonyl, Alkoxysulfonyl, Cyano, Trifluormethyl, Sulfo, eine Gruppe der Formel -COOY₁, -CONY₁Y₂ oder-SO₂NY₁Y₂, worin Y₁ und Y₂ wie oben definiert sind, oder eine Gruppe der Formel 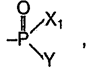 worin X₁ und Y wie oben definiert sind, und R4" Wasserstoff oder unsubstituiertes oder nicht-chromophor substituiertes Alkyl bedeuten.

4. 4-Styryl-4'-vinylbiphenyle nach Anspruch 3 der Formel 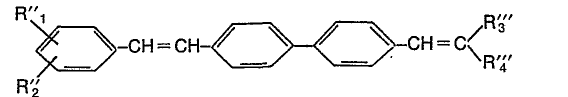 worin
R₁" Wasserstoff, Halogen, C₁-C₄-Alkyl, C₁-C₄-Alkoxy, C₁-C₄-Alkylsulfonyl, Cyano, gegebenenfalls durch Chlor, Methyl oder/und Methoxy substituiertes Phenyl oder Phenylsulfonyl, eine Gruppe der Formel
-COOY₁', -CONY₁'Y₂' oder -SO₂NY₁'Y₂' , worin Yᵢ' für Wasserstoff, C₁-C₈-Alkyl, C₃-C₄-Alkenyl, Cyclohexyl, C₂-C₄-Hydroxyalkyl, C₃-C₆-Alkoxyalkyl, einen Rest der Formel
-(-CH₂CH₂-O-)ₙ-(-C₁-C₄-Alkyl), worin n eine ganze Zahl zwischen 1 und 6 ist, C₆-C₉-Phenoxyalkyl, C₂-C₆-Carboxyalkyl, C₃-C₆-Carbalkoxyalkyl, C₂ C₅-Cyanoalkyl, unsubstituiertes oder durch Chlor, Methyl oder/und Methoxy substituiertes Phenyl oder Benzyl, C₃-C₇-Dialkylaminoalkyl oder Phenäthyl, Y₂' für Wasserstoff, C₁-C₄-Alkyl, C3-C4-Alkenyl oder C₂-C₄-Hydroxyalkyl oder Y₁' und Y2 zusammen mit dem Stickstoffatom, an das sie gebunden sind, für einen 5- oder 6-gliedrigen gesättigten heterocyclischen Ring stehen, der noch ein weiteres Stickstoff- oder Sauerstoffatom als Ringglied enthalten kann und der gegebenenfalls durch 1 oder 2 C₁-C₄-Alkylgruppen substituiert sein kann, oder worin Y₁' in der Gruppe -COOY₁' zusätzlich zu den vorstehenden Bedeutungen noch für ein Alkalimetall- oder Ammoniumion stehen kann; oder eine Gruppe der Formel 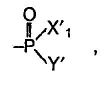 worin X₁' und Y' unabhängig voneinander für C₁-C₄-Alkyl, Benzyl, unsubstituiertes oder durch Chlor, Methyl oder/und Methoxy substituiertes Phenyl stehen,
R2" Wasserstoff, Halogen oder C₁-C₄-Alkyl,
R₃"' Cyano, C₁-C₄-Alkylsulfonyl oder eine Gruppe der Formel -COOY₁', -CONY₁'Y₂ , -SO₂NY₁'Y₂' oder 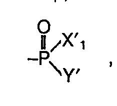 worin Y₁', Y₂', X₁' und Y' wie oben definiert sind und
R₄'" Wasserstoff oder Cᵢ-C₄-Alkyl bedeuten.

5. 4-Styryl-4'-vinylbiphenyle nach Anspruch 4 der Formel 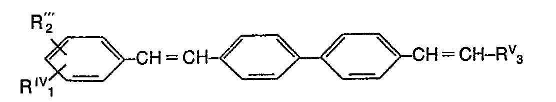 worin R₁^{IV} C₁-C₄-Alkylsulfonyl, Cyano, 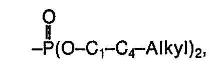 oder -COOY₁"', worin Y₁'" für
C₁-C₄-Alkyl, C₂-C₄-Hydroxyalkyl,
C₃-C₆-Alkoxyalkyl,
(-CH₂-CH₂-O-)ₙ-(-C₁-C₄-Alkyl), worin n' eine ganze Zahl zwischen 1 und 4 ist, C₃-C₆-Carbalkoxyalkyl, C₂-C₆-Carboxyalkyl oder C₂C₅-Cyanoalkyl steht, R₂"' Wasserstoff oder Halogen und
R₃^{V} C₁-C₄-Alkylsulfonyl, Cyano, 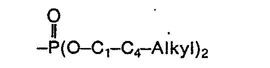 oder -COOY₁"', worin Y₁"' wie oben definiert ist, bedeuten.

6. 4-Styryl-4'-vinylbiphenyle nach Anspruch 5 der Formel 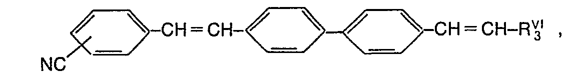 worin
R3^{IV} Cyano oder -COOY₁^{IV} bedeutet, worin Y₁^{IV} für C₁-C₄-Alkyl, C₂-C₄-Hydroxyalkyl, C₃-C₆-Alkoxyalkyl oder (-CH₂-CH₂-O-)ₙ-(-C₁-C₄-Alkyl) steht, wobei n' eine ganze Zahl zwischen 1 und 4 ist.

7. 4-Styryl-4'-vinylbiphenyl nach Anspruch 6 der Formel

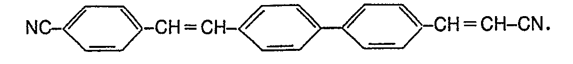

8. Verfahren zur Herstellung eines in Anspruch 1 definierten 4-Styryl-4'-vinylbiphenyls, dadurch gekennzeichnet, dass man einen Biphenyl-4,4'- dialdehyd der Formel 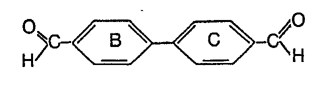 mit einer Verbindung der Formel 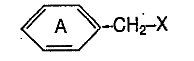 zu einem Aldehyd der Formel 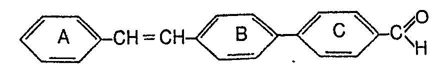 umsetzt und diesen Aldehyd dann weiter mit einer Verbindung der Formel 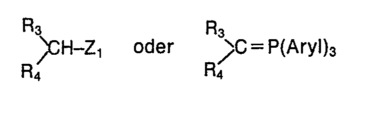 zu einer in Anspruch 1 definierten Verbindung umsetzt, wobei A, B, C, R₃ und R₄ die in Anspruch 1 angegebenen Bedeutungen haben und wobei X und Z₁ gleich oder verschieden sind und unabhängig voneinander Wasserstoff oder einen Rest der Formel -COOZ, worin Z für Wasserstoff oder Alkyl steht, 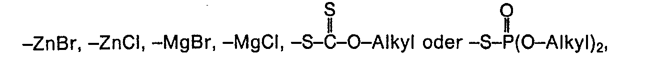 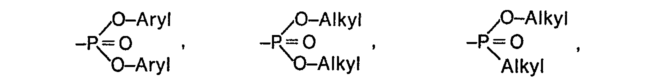 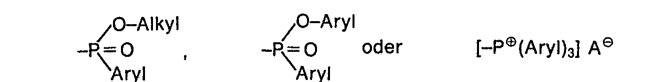 bedeutet, wobei A⊖ für ein einwertiges farbloses Anion steht.

9. Verfahren nach Anspruch 8, dadurch gekennzeichnet, dass X und Z₁ unabhängig voneinander eine Gruppe der Formel 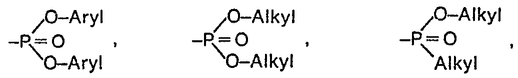 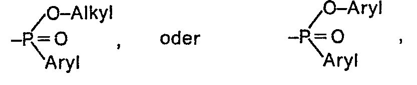 bedeuten.

10. Verfahren nach einem der Ansprüche 8 oder 9, dadurch gekennzeichnet, dass man einen Biphenyl-4,4'-dialdehyd der Formel 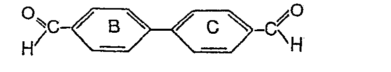 mit einer Verbindung der Formel 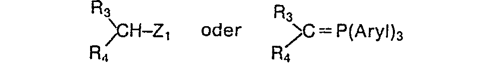 zu einem Aldehyd der 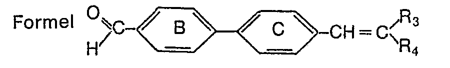 und diesen mit einer Verbindung der Formel 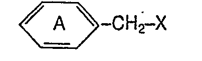 zu einer der im Anspruch 1 definierten Verbindungen umsetzt.

11. Verfahren nach einem der Ansprüche 8 bis 10, dadurch gekennzeichnet, dass man die Umsetzung eines Biphenyl-4,4'-dialdehyds mit einer Verbindung der Formel 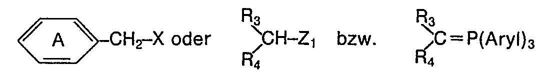 in einem Lösungsmittel oder Lösungsmittelgemisch vornimmt, in welchem der entstehende Monoaldehyd schwer löslich ist und auskristallisiert, und in Gegenwart eines alkalischen Kondensationsmittels durchführt.

12. Verfahren nach Anspruch 11, dadurch gekennzeichnet, dass man die Umsetzung in Gegenwart eines Alkalimetallalkoholats bei Temperaturen zwischen 0 und 50 °C durchführt.

13. Verfahren nach einem der Ansprüche 8 bis 12, dadurch gekennzeichnet, dass man den in erster Stufe erhaltenen Monoaldehyd ohne Isolierung weiter umsetzt.

14. Verfahren nach einem der Ansprüche 8 bis 12, dadurch gekennzeichnet, dass man den in erster Stufe erhaltenen Monoaldehyd isoliert und dann weiter umsetzt.

15. Verfahren nach Anspruch 14, dadurch gekennzeichnet, dass man die Umsetzung des in erster Stufe erhaltenen Monoaldehyds mit einer Verbindung der Formel 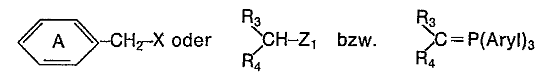 in Gegenwart eines alkalischen Kondensationsmittels durchführt.

16. Verfahren nach Anspruch 15, dadurch gekennzeichnet, dass man die Umsetzung in Gegenwart eines Alkalimetallalkoholates bei Temperaturen zwischen 20 und 100 °C durchführt.

17. Mittel zum optischen Aufhellen von hochmolekularen organischen Materialien, dadurch gekennzeichnet, dass es eine oder mehrere der in den Ansprüchen 1 bis 7 definierten Verbindungen enthält.

18. Mittel nach Anspruch 17, dadurch gekennzeichnet, dass es neben einem auf dem behandelten Substrat eine rötliche Nuance hervorrufenden der in den Ansprüchen 1 bis 7 definierten optischen Aufheller zusätzlich noch einen optischen Aufheller enthält, der auf dem behandelten Substrat eine grünliche bis bläuliche Nuance hervorruft.

19. Mittel nach Anspruch 18, dadurch gekennzeichnet, dass es einen in den Ansprüchen 1 bis 7 definierten optischen Aufheller und einen oder mehrere optische Aufheller aus der Klasse der Bis-styrylbenzole, Benzoxazolylstilbene, 4,4'-Divinylstilbene, Naphthalimide, 4,4'-Bis-styrylbiphenyle, 4,4'-Bis-triazolylstilbene, Bis-benzoxazolyl-thiophene, Naphthotriazolyl-stilbene oder der Cumarine enthält.

20. Hochmolekulares organisches Material, enthaltend 0,001 bis 2 Gew.-% eines der in den Ansprüchen 1 bis 7 definierten optischen Aufheller oder einer der in den Ansprüchen 17 bis 19 definierten Aufhellermischungen.

21. Verwendung von in den Ansprüchen 1 bis 7 definierten Verbindungen zum optischen Aufhellen von natürlichen, halbsynthetischen oder synthetischen hochmolekularen organischen Materialien.

22. Verwendung nach Anspruch 21 zum optischen Aufhellen von Polvesterfasern.

## Claims

1. A 4-styryl-4'-vinylbiphenyl of the formula 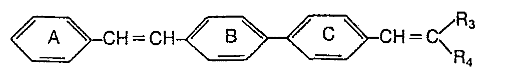 wherein the benzene rings A, B and C can carry non-chromophoric substituents and R₃ is a second order non-chromophoric substituent, and R₄ is hydrogen or a non-chromophoric substituent which cannot be a second order substituent.

2. A 4-styryl-4'-vinylbiphenyl according to claim 1 of the formula 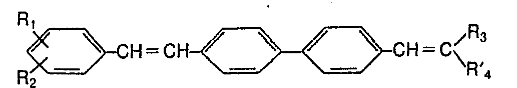 wherein each of R₁ and R₂ is hydrogen or a non-chromophoric substituent, or both together in the ortho-position complete a fused ring, R₃ is a second order non-chromophoric substituent and R₄' is hydrogen or alkyl or alkenyl, each unsubstituted or substituted by non-chromophoric groups.

3. A 4-styryl-4'-vinylbiphenyl according to claim 2 of the formula 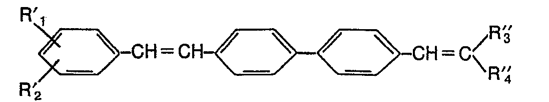 wherein R₁' is hydrogen or halogen; alkyl, alkoxy or alkylsulfonyl, each unsubstituted or substituted by non-chromophoric groups; phenyl, phenylalkyl or phenylsulfonyl, phenoxy or phenylalkoxy, each unsubstituted or substituted by non-chromophoric groups; or is cyano, a group of the formula -COOY₁,-CONY₁Y₂Or-SO₂NY₁Y₂, wherein each of Y₁ and Y₂ independently is hydrogen, alkenyl, propargyl, cycloalkyl containing 5 or 6 ring carbon atoms, or is alkyl, phenyl or phenylalkyl, each unsubstituted or substituted by non-chromophoric groups; or Y₁ and Y₂, together with the nitrogen atom to which they are attached, are a 5- or 6- membered saturated heterocyclic ring which can additionally contain 1 or 2 hetero-atoms as ring members and can also be substituted by alkyl groups; or wherein Y₁ in the group -COOY₁ can also additionally be a salt-forming cation; or R₁' is a group of the formula 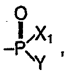 wherein each of X₁ and Y independently of the other is halogen, alkyl, alkenyl, phenyl, phenylalkyl, hydroxy, alkoxy, phenylalkoxy, cycloalkoxy, phenoxy, amino, mono- or dialkylamino, phenylalkylamino, acylamino, phenylamino, cycloalkylamino, morpholino, piperidino or pyrrolidino; or R₁' together with R₂' in the ortho-position are the radical of the formula -CH = CH-CH = CH-, -O-CH₂-O- or -0-CH_{z}-CH_{z}-O-; R₂' is hydrogen, halogen, or alkyl or alkoxy, each unsubstituted or substituted by non-chromophoric groups; or R₂' together with R₁' in the ortho-position are the radical of the formula -CH=CH-CH=CH-, -O-CH_{z}-O- or -O-CH₂-CH₂-O-; R₃" is alkylsulfonyl, phenylsulfonyl, alkoxysulfonyl, cyano, trifluoromethyl, sulfo, a group of the formula -COOY₁, -CONY₁Y₂ or -SO₂NV₁Y₂, wherein Y₁ and Y₂ are as defined above; or is a group of the formula 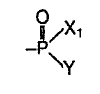 wherein X₁ and Y are as defined above; and R4" is hydrogen or alkyl which is unsubstituted or substituted by non-chromophoric groups.

4. A 4-styryl-4'-vinylbiphenyl according to claim 3 of the formula 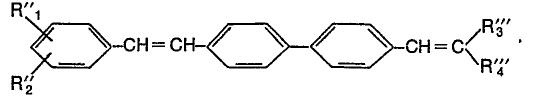 wherein R₁" is hydrogen, halogen, C₁-C₄alkyl, C₁-C₄alkoxy, C₁-C₄alkylsulfonyl, cyano, or phenyl or phenylsulfonyl, each unsubstituted or substituted by chlorine, methyl and/or methoxy; or is a group of the formula -COOY₁', -CONY₁'Y₂' or -SO₂NY₁'Y₂, wherein Y₁' is hydrogen, C₁-C₈alkyl, C₃-C₄alkenyl, cyclohexyl, C₂-C₄hydroxyalkyl, C₃-C₆alkoxyalkyl, a radical of the formula -(-CH₂CH₂O-)ₙ-(-C₁-C₄alkyl), wherein n is an integer from 1 to 6, or is C₆-C₉phenoxy- alkyl, C₂-C₆carboxyalkyl, C₃-C₆carbalkoxyalkyl, C₂-C₅cyanoalkyl, phenyl or benzyl, each unsubstituted or substituted by chlorine, methyl and/or methoxy, or is C3-C7dialkylaminoalkyl or phenethyl; Y₂' is hydrogen, C₁-C₄alkyl, C₃-C₄alkenyl or C₂-C₄hydroxyalkyl, or Y₁' and Y2', together with the nitrogen atom to which they are attached, are a 5- or 6-membered saturated heterocyclic ring which can additionally contain a further nitrogen or oxygen atom as ring member and which can be substituted by 1 or 2 C₁-C₄alkyl groups; or wherein Y₁' in the group -COOY₁' can additionally be an alkali metal ion or an ammonium ion; or R₁" is a group of the formula 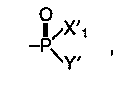 , wherein each of X₁' and Y' independently of the other is C₁-C₄alkyl, benzyl, phenyl which is unsubstituted or substituted by chlorine, methyl and/or methoxy; R2" is hydrogen, halogen or C₁-C₄alkyl; R₃"' is cyano, C₁-C₄alkylsulfonyl or a group of the formula -COOY₁', -CONY₁'Y₂' , -SO₂NY₁'Y₂' or 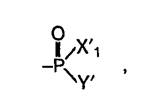 wherein Y₁', Y₂', X₁' and Y' are as defined above; and R₄"' is hydrogen or C₁-C₄alkyl.

5. A 4-styryl-4'-vinylbiphenyl according to claim 4 of the formula 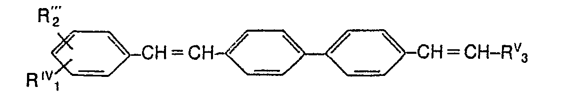 wherein R₁^{IV} is C₁-C₄alkylsulfonyl, cyano, 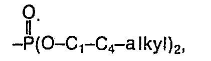 or -COOY₁'", wherein Y₁'",
is C₁-C₄alkyl, C₂-C₄hydroxyalkyl, C₃-C₆alkoxyalkyl,-(-CH₂-CH₂-O-)ₙ-(-C₁-C₄alkyl), wherein n' is integer from 1 to 4, or is C₃-C₆carbalkoxyalkyl, C₂-C₆carboxyalkyl or C₂-C₅cyanoalkyl; R₂'" is hydrogen or halogen; and R₃^{V} is C₁-C₄alkylsulfonyl, cyano, 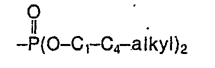 or -COOY₁"', wherein Y₁"' is as defined above.

6. A 4-styryl-4'-vinylbiphenyl according to claim 5 of the formula 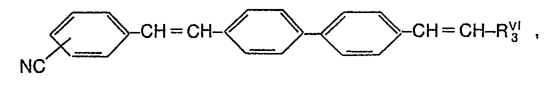 wherein R₃^{IV} is cyano or-COOY₁^{IV}, in which Y₁^{IV} is C₁-C₄alkyl, C₂-C₄hydroxyalkyl, C₃-C₆alkoxyalkyl or (-CH₂-CH₂O-)ₙ'-(-C₁-C₄alkyl), wherein n' is an integer from 1 to 4.

7. The 4-styryl-4'-vinylbiphenyl according to claim 6 of the formula

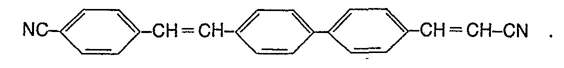

8. A process for the production of a 4-styryl-4'- vinylbiphenyl as defined in claim 1, which process comprises reacting a biphenyl-4,4'-dialdehyde of the formula 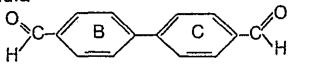 with a compound of the formula 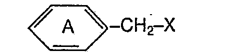 to give an aldehyde of the formula 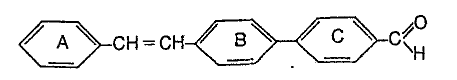 and subsequently reacting this aldehyde with a compound of the formula 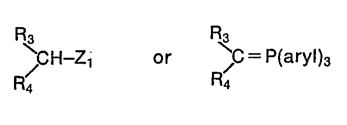 to give a compound as defined in claim 1, in which formulae above A, B, C, R₃ and R₄ are as defined in claim 1, and X and Z₁ are the same or different and each independently of the other is hydrogen or a radical of the formula -COOZ, wherein Z is hydrogen or alkyl, 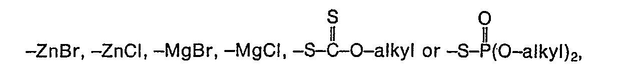 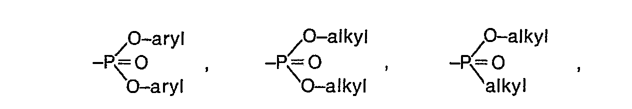 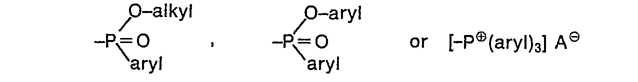 wherein A^{⊖} is a monovalent colourless anion.

9. A process according to claim 8, wherein each of X and Z₁ independently of the other is a group of the formula

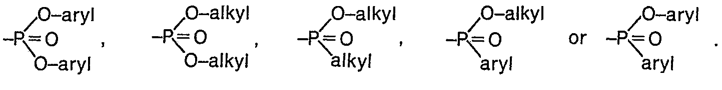

10. A process according to either of claims 8 or 9, which process comprises reacting a biphenyl-4,4'-dialdehyde of the formula 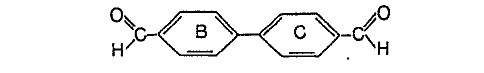 with a compound of the formula 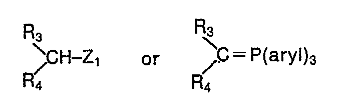 to give an aldehyde of the 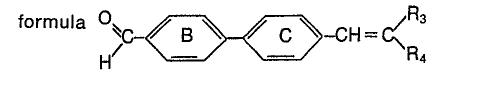 and subsequently reacting this aldehyde with a compound of the formula 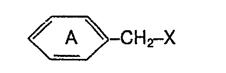 to give a compound as defined in claim 1.

11. A process according to any of claims 8 to 10, wherein the reaction of a biphenyl-4,4'-dialdehyde with a compound of the formula 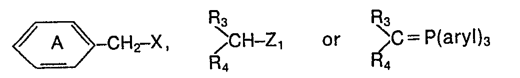 is carried out in a solvent in which the resultant monoaldehyde is sparingly soluble and from which it crystallises out, and in the presence of an alkaline condensing agent.

12. A process according to claim 11, wherein the reaction is carried out in the presence of an alkali metal alcoholate in the temperature range from 0° to 50 °C.

13. A process according to any one of claims 8 to 12, wherein the monoaldehyde obtained in the first step is further reacted without being isolated.

14. A process according to any one of claims 8 to 12, wherein the monoaldehyde obtained in the first step is isolated and then further reacted.

15. A process according to claim 14, wherein the reaction of the monoaldehyde obtained in the first step with a compound of the formula 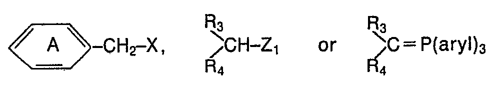 is carried out in the presence of an alkaline condensing agent.

16. A process according to claim 15, wherein the reaction is carried out in the presence of an alkali metal alcoholate in the temperature range from 20° to 100 °C.

17. A composition for whitening organic material of high molecular weight, which composition contains one or more compounds as defined in claims 1 to 7.

18. A composition according to claim 17 which, in addition to containing a fluorescent whitening agent as defined in any one of claims 1 to 7 which gives a reddish hue on the treated substrate, also contains a fluorescent whitening agent which gives a greenish to bluish hue on said substrate.

19. A composition according to claim 18 which contains a fluorescent whitening agent as defined in any one of claims 1 to 7 and, in addition, one or more fluorescent whitening agents of the class of the bis-styrylbenzenes, benzoxazolylstilbenes, 4,4'-divinylstilbenes, naphthalimides, 4,4'-bis- styrylbiphenyls, 4,4'-bis-triazolylstilbenes, bis- benzoxazolylthiophenes, naphthotriazolylstil- benes or coumarins.

20. Organic material of high molecular weight containing 0.001 to 2% by weight of a fluorescent whitening agent as defined in any one of claims 1 to 7, or of a mixture of fluorescent whitening agents as defined in any one of claims 17 to 19.

21. Use of a compound as defined in any one of claims 1 to 7 for whitening natural, semi-synthetic or synthetic organic materials of high molecular weight.

22. Use according to claim 21 for whitening polyester fibres.

## Revendications

1. 4-styryl-4'-vinylbiphényles de formule 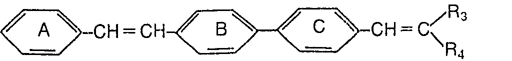 dans laquelle les noyaux benzéniques A, B et C peuvent être substitués par des substituants non chromophores, R₃ représente un substituant non chromophore de second ordre et R₄ représente un hydrogène ou un substituant non chromophore qui, cependant, ne peut pas être de second ordre.

2. 4-styryl-4'-vinylbiphényles selon la revendication 1, de formule 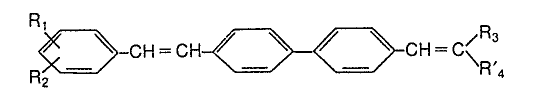 dans laquelle R₁ et R₂ représentent chacun un hydrogène ou un substituant non chromophore ou les deux ensemble en position ortho représentent le complément à un cycle condensé, R₃ représente un substituant non chromophore de second ordre et R₄' représente un hydrogène ou un reste alkyle ou alcényle éventuellement substitué par des substituants non chromophores.

3. 4-styryl-4'-vinylbiphényles selon la revendication 1, de formule 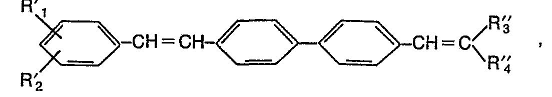 dans laquelle R₁' représente un hydrogène, un halogène, un reste alkyle, alcoxy ou alkylsulfonyle non substitué ou portant des substituants non chromophores, un reste phényle, phénylalkyle ou phénylsulfonyle non substitué ou portant des substituants non chromophores, le reste phenoxy, un reste phénylalcoxy, le reste cyano, un groupe de formule -COOY₁, -CONY₁Y₂ ou -S0₂NY₁Y₂, dans laquelle Y₁ et Y₂ représentent, indépendamment l'un de l'autre, un hydrogène, un reste alcényle, le reste propargyle, un reste cycloalkyle ayant 5 ou 6 atomes de carbone dans le cycle, un reste alkyle non substitué ou portant des substituants non chromophores, le reste phényle, un reste phénylalkyle, ou bien Y₁ et Y₂ représentent ensemble avec l'atome d'azote auquel ils sont liés un cycle hétérocyclique saturé de 5 à 6 chaînons, qui peut contenir en plus 1 ou 2 hétéroatomes comme membres du cycle et qui peut être substitué par des groupes alkyle, ou bien dans laquelle Y₁ dans le groupe -COOY₁ peut représenter en outre un cation formant un sel, ou bien un groupe de formule 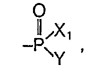 dans lequel X₁ et Y représentent, indépendamment l'un de l'autre, un halogène, les restes alkyle, alcényle, phényle, phénylalkyle, hydroxy, alcoxy, phénylalcoxy, cycloalcoxy, phénoxy, amino, mono- ou dialkylamino, phénylalkyl- amino, acylamino, phénylamino, cycloalkylamino, morpholino, pipéridino ou pyrrolidino; ou forme avec R2 se trouvant en position ortho le reste de formule -CH=CH-CH=CH-, -O-CH₂-O- ou -O-CH₂-CH₂-O-; R2 représente un hydrogène, un halogène, un reste alkyle ou alcoxy non substitué ou portant des substituants non chromophores, ou bien forme avec R₁' se trouvant en position ortho le reste de formule -CH=CH-CH=CH-, -O-CH_{z}-O-, ou O-CH₂-CH₂-O-; R3" représente un reste alkylsulfonyle, le reste phénylsulfonyle, un reste alcoxysulfonyle, le reste cyano, le reste trifluorométhyle, le reste sulfo, un groupe de formule -COOY₁, -CONY₁Y₂ ou -SO₂NY₁Y₂, dans lequel Y₁ et Y₂ sont tels que définis ci-dessus, ou bien un groupe de formule 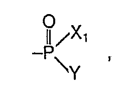 dans laquelle X₁ et Y sont tels que définis ci-dessus; et R4" représente un hydrogène ou un reste alkyle non substitué ou portant des substituants non chromophores.

4. 4-styryl-4'-vinylbiphényles selon la revendication 3, de formule 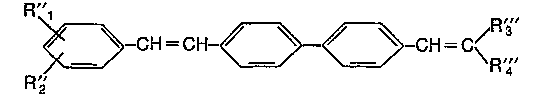 dans laquelle
R₁" représente un hydrogène, un halogène, un reste alkyle en C₁-C₄, un reste alcoxy en C₁-C₄, un reste (alkyl en C₁-C₄)sulfonyle, un reste cyano, un reste phényle ou phénylsulfonyle portant éventuellement des substituants chlore, méthyle et/ou méthoxy, un groupe de formule -COOY₁', -CONY₁'Y₂' ou -SO₂NY₁'Y₂', dans laquelle Y₁' représente un hydrogène, un reste alkyle en C₁-C₈, un reste alcényle en C₃-C₄, un reste cyclohexyle, un reste hydroxyalkyle en C₂-C₄, un reste alcoxyalkyle en C₃-C₆, un reste de formule -(-CH2CH2-O-)n-(-alkyle en C₁-C₄), dans laquelle n représente un nombre entier compris entre 1 et 6, un reste phénoxyalkyle en C₆-C₉, un reste car- boxyalkyle en C₂-C₆, un reste carbalcoxyalkyle en C₃-C₆, un reste cyanoalkyle en C₂-C₅, un reste phényle ou benzyle non substitué ou à substituants chlore, méthyle et/ou méthoxy, un reste dialkylaminoalkyle en C₃-C₇ ou un reste phéné- thyle, Y2 représente un hydrogène, un reste alkyle en C₁-C₄, un reste alcényle en C₃-C₄ ou un reste hydroxyalkyle en C₂-C₄ ou bien Y₁' et Y₂' représentent ensemble avec l'atome d'azote auquel ils sont liés un cycle hétérocyclique saturé de 5 ou 6 chaînons, qui peut contenir encore un autre atome d'azote ou d'oxygène comme membre du cycle et qui peut être substitué éventuellement par 1 ou 2 groupes alkyle en C₁-C₄, ou bien Y₁' dans le groupe-COOY₁', en plus des significations précédentes, représente encore un ion de métal alcalin ou ammonium; ou bien le groupe de formule 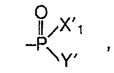 dans lequel X₁' et Y' représentent, indépendamment l'un de l'autre, un reste alkyle en C₁-C₄, le reste benzyle, un reste phényle non substitué ou à substituants chlore, méthyle et/ou méthoxy;
R2" représente un hydrogène, un halogène ou un reste alkyle en C₁-C₄;
R₃'" représente le reste cyano, un reste (alkyle en C₁-C₄)sulfonyle ou un groupe de formule -COOY₁', -CONY₁'Y₂' , -SO₂NY₁'Y₂' ou 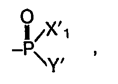 dans laquelle Y₁', Y₂', X₁' et Y' sont tels que définis ci-dessus et
R₄"' représente un hydrogène ou un reste alkyle en C₁-C₄.

5. 4-styryl-4'-vinylbiphényles selon la revendication 4 de formule 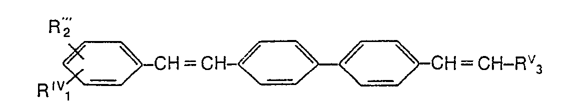 dans laquelle R₁^{IV} représente un reste (alkyle en C₁-C₄)sulfonyle, le reste cyano, un reste 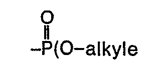 en C₁-C₄))₂ ou -COOY₁"',
où Y₁"' représente un reste alkyle en C₁-C₄, un reste hydroxyalkyle en C_{z}-C₄, un reste alcoxyalkyle en C₃-C₆, (-CH₂-CH₂-O-)ₙ-(-alkyle en C₁-C₄) où n' est un nombre entier compris entre 1 et 4, un reste carbalcoxyalkyle en C₃-C₆, un reste car- boxyalkyle en C₂-C₆ ou un reste cyanoalkyle en C₂-C₅, R₂"' représente un hydrogène ou un halogène et
R₃^{IV} représente un reste (alkyle en Ci-C₄)sulfo- nyle, le reste cyano, un reste 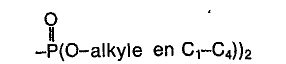 ou -COOY₁"', où Y₁"' est tel que défini ci-dessus.

6. 4-styryl-4'-vinylbiphényles selon la revendication 5 de formule 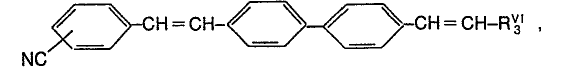 dans laquelle
R₃^{1v} représente un reste cyano ou -COOY₁^{IV}, où Y₁^{IV} représente un reste alkyle en C₁-C₄, un reste hydroxyalkyle en C₂-C₄, un reste alcoxyalkyle en C₃-C₆ ou (-CH₂-CH₂O-)ₙ-(-alkyle en C₁-C₄), où n' représente un nombre entier compris entre 1 et 4.

7. 4-styryl-4'-vinylbiphényle selon la revendication 6 de formule

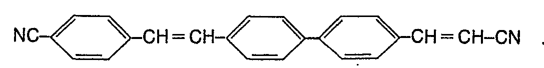

8. Procédé de préparation d'un 4-styryl-4'-vinyl- biphényle défini dans la revendication 1, caractérisé par le fait que l'on transforme un biphényl-4,4'-dialdéhyde de formule 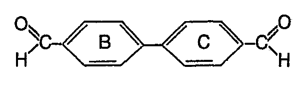 par réaction avec un composé de formule 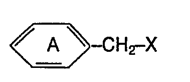 en un aldéhyde de formule 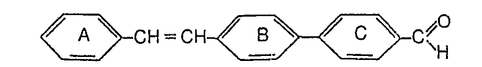 et que l'on transforme ensuite cet aldéhyde par réaction avec un composé de formule 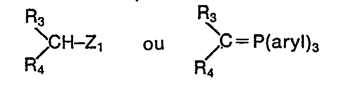 en un composé défini dans la revendication 1, A, B, C, R₃ et R₄ ayant les significations indiquées dans la revendication 1 et X et Z₁ étant identiques ou différents et représentant indépendamment l'un de l'autre un hydrogène ou un reste de formule -COOZ, dans lequel Z représente un hydrogène ou un reste alkyle, 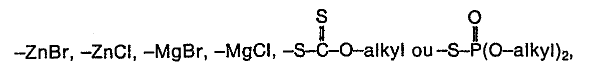 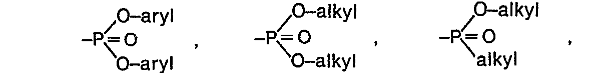 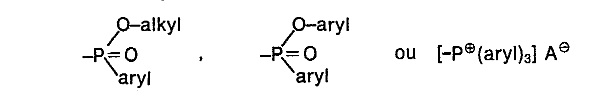 où A⁹ représente un anion incolore monovalent.

9. Procédé selon la revendication 8, caractérisé par le fait que X et Z₁ représentent indépendamment l'un de l'autre un groupe de formule

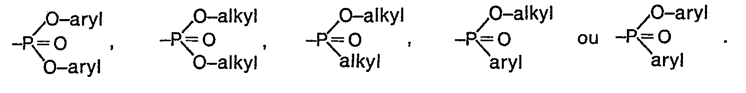

10. Procédé selon une des revendications 8 ou 9, caractérisé par le fait que l'on transforme un biphényl-4,4'-dialdéhyde de formule 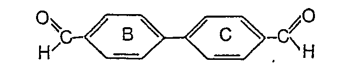 par réaction avec un composé de formule 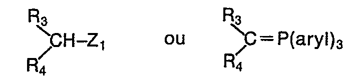 en un aldéhyde de formule 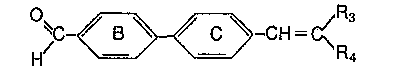 et que l'on transforme celui-ci par réaction avec un composé de formule 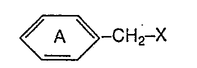 en un des composés définis dans la revendication 1.

11. Procédé selon une des revendications 8 à 10, caractérisé par le fait que l'on réalise la réaction d'un biphényl-4,4'-dialdéhyde avec un composé de formule 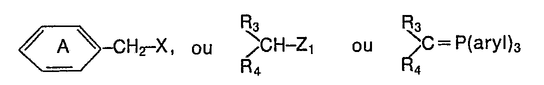 dans un solvant ou un mélange de solvants, dans lequel le monoaldéhyde qui se forme est difficilement soluble et cristallise, et que l'on effectue la réaction en présence d'un agent de condensation - alcalin.

12. Procédé selon la revendication 11, caractérisé par le fait que l'on réalise la réaction en présence d'un alcoolate de métal alcalin à des températures comprises entre 0 et 50 °C.

13. Procédé selon une des revendications 8 à 12, caractérisé par le fait que l'on fait réagir le monoaldéhyde obtenu dans la première étape sans l'isoler.

14. Procédé selon une des revendications 8 à 12, caractérisé par le fait que l'on isole le monoaldéhyde obtenu dans la première étape et qu'ensuite, on le fait de nouveau réagir.

15. Procédé selon la revendication 14, caractérisé par le fait que l'on effectue la réaction du monoaldéhyde obtenu dans la première étape avec un composé de formule 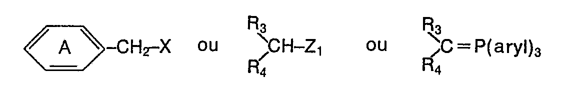 en présence d'un agent de condensation alcalin.

16. Procédé selon la revendication 15, caractérisé par le fait que l'on effectue la réaction en présence d'un alcoolate de métal alcalin à des températures comprises entre 20 et 100 °C.

17. Agent pour l'azurage optique de matières organiques de poids moléculaire élevé, caractérisé par le fait qu'il contient un ou plusieurs des composés définis dans les revendications 1 à 7.

18. Agent selon la revendication 17, caractérisé par le fait qu'il contient encore, à côté de l'un des azurants optiques définis dans les revendications 1 à 7, donnant une teinte rougeâtre sur le substrat traité, un azurant optique qui donne sur le substrat traité une teine verdâtre à bleuâtre.

19. Agent selon la revendication 18, caractérisé par le fait qu'il contient un azurant optique défini dans les revendications 1 à 7 et un ou plusieurs azurants optiques appartenant au groupe comprenant les bis-styrylbenzènes, les benzoxazolylstil- bènes, les 4,4'-divinylstilbènes, les naphtalimi- des, les 4,4'-bis-styrylbiphényles, les 4,4'-bis-tri- azolylstilbènes, les bis-benzoxazolyl-thiophènes, les naphtotriazolyl-stilbènes et les coumarines.

20. Matière organique de poids moléculaire élevé, contenant de 0,001 à 2% en poids d'un des azurants optiques définis dans les revendications 1 à 7 ou un des mélanges d'azurants définis dans les revendications 17 à 19.

21. Utilisation des composés définis dans les revendications 1 à 7 pour l'azurage optique des matières organiques naturelles, semi-synthétiques ou synthétiques de poids moléculaire élevé.

22. Utilisation selon la revendication 21 pour l'azurage optique des fibres de polyamide.
